# EUROPEAN PATENT APPLICATION

(11) **EP 2 944 961 A1**
(43) Date of publication of application: **18.11.2015**
(21) Application number: 15172438.2
(22) Date of filing: 03.05.2012
(51) Int. Cl.: G01N 33/574, C12Q 1/68

(54) **MARKERS FOR CANCER PROGNOSIS AND THERAPY AND METHODS OF USE**

(30) Priority: 06.05.2011 US 201161483410 P
(62) Divisional of application: 12727927.1
(71) Applicant: XenTech, 91000 Evry (FR)
(72) Inventor: JUDDE, Jean-Gabriel, 94110 Arcueil (FR); LEGRIER, Marie-Emmanuelle, 75005 Paris (FR); CAIRO, Stefano, 91310 Longpont sur Orge (FR)
(74) Representative: Santarelli

(57) **Abstract**

The invention relates generally to the field of cancer prognosis and treatment. More particularly, the present invention relates to methods and compositions that utilize a particular panel of gene products ("biomarkers") and their differential expression patterns ("expression signatures"), wherein the expression patterns correlate with responsiveness, or lack thereof, to chemotherapy treatment. The invention is based on the identification of a specific set of biomarkers that are differentially expressed in chemotherapy-treated tumors and which are useful in predicting the likelihood of a therapeutic response, including residual disease persistence and subsequent tumor recurrence in cancer patients receiving chemotherapy. The gene panel is also useful in designing specific adjuvant modalities with improved therapeutic efficiency. Also disclosed are methods for characterizing tumors according to expression of the biomarkers described herein.

## Description

### 1. INTRODUCTION

The invention relates generally to the field of cancer prognosis and treatment. More particularly, the present invention relates to methods and compositions that utilize a particular panel of gene products ("biomarkers") and their differential expression patterns ("expression signatures"), wherein the expression patterns predict responsiveness, or lack thereof, to chemotherapy treatment. The invention is based on the identification of a specific set of biomarkers that are differentially expressed in chemotherapy-treated tumors and which are useful in predicting the likelihood of a therapeutic response, including residual disease persistence and subsequent tumor recurrence in cancer patients receiving chemotherapy. The gene panel is also useful in designing specific adjuvant modalities with improved therapeutic efficacy. Also disclosed are methods for characterizing tumors according to expression of the biomarkers described herein.

### 2. BACKGROUND OF INVENTION

Although progress has been made in the field of cancer treatment, most currently available cancer treatments fail in providing complete tumor eradication. This partial efficacy is largely due to innate or acquired resistance of cancer cells to anticancer drug therapies and is a major factor in disease relapse and treatment failure. Therefore, it is important to investigate the molecular basis of tumor resistance to treatment and identify genes or pathways to be targeted to overcome drug resistance in order to improve the efficacy of therapeutic intervention. The heterogeneous nature of cancer makes this identification very difficult.

Modem molecular biology and biochemistry have revealed hundreds of genes whose activities influence the behavior of tumor cells, their state of differentiation, and their sensitivity or resistance to certain therapeutic drugs. However, with a few exceptions, the status of these genes has not been exploited for the purpose of routinely making clinical decisions about drug treatments. One notable exception is the use of estrogen receptor (ER) and/or progesterone receptor (PR) protein expression in breast carcinomas to select patients to treatment with anti-estrogen drugs. Another exceptional example is the use of ErbB2 (Her2) protein expression in breast carcinomas to select patients to treatment with the Her2 antagonist drug Herceptin®.

Triple-negative/basal-like breast cancer (TNBC/BLBC) comprises 15-20% of all breast cancers. They are often more undifferentiated, carry an increased risk of distant metastasis, tend to relapse early and have been associated with a short post-recurrence survival. TNBCs lack hormonal receptors and Her2 overexpression and are therefore not candidate for anti-oestrogen or Herceptin® therapy (Linn SC. and Van't Veer L. Eur. J Cancer 45, Suppl 1: 11-26, 2009). Today's conventional treatment of TNBC patients is thus based on combinations of cytotoxic drugs, including anthracyclins, cyclophosphamide, 5-fluorouracil and taxanes. Within the TNBC subtype, neither prognostic nor predictive factors are currently available to guide the choice of the most effective chemotherapies. One exception concerns a sub-population of TNBC or ovarian tumors with BRCA1 pathway dysfunction, which results into a defect in repair of DNA double strand breaks (DSB) and confers such tumors increased sensitivity to chemotherapeutic agents inducing DNA double-strand breaks (DSB) such as bifunctional alkylators and platinum agents. In addition, BRAC1-deficient tumors are sensitive to blockade of repair of DNA single-strand breaks (SSB) through the inhibition of PARP1 (Linn SC. and Van't Veer L. Eur. J Cancer 45, Suppl 1: 11-26, 2009). Consequently, inappropriate treatment in the adjuvant setting is common for TNBC, and there is an urgent need to develop novel and better targeted therapeutic approaches.

Breast cancer neoadjuvant chemotherapy and residual disease. Preoperative (neoadjuvant) systemic chemotherapy is generally proposed to patients with advanced/infiltrating breast carcinoma. Following neoadjuvant chemotherapy, pathological complete response (pCR) is defined as no microscopic evidence of residual cancer in the breast and regional lymph nodes at the time of surgical resection. Presently, pCR is the best surrogate marker for favorable long-term outcome in BC patients, corresponding to a 3-year overall survival (OS) of 90-100%. However, a major problem today is that over 70% of patients receiving preoperative chemotherapy do not achieve pCR and have residual disease at the time of surgical resection. This group of patients has a poor outcome, with a 3-year OS of only 60-70% in spite of receiving adjuvant chemotherapy (Linn SC. and Van't Veer L. Eur. J Cancer 45, Suppl 1: 11-26, 2009).

The presence of residual disease in the majority of cancer patients who have received preoperative chemotherapy indicates the persistence of a sub-population of chemo-resistant cells responsible for treatment failure. Understanding the mechanisms underlying their persistence may lead to the development of novel and more efficient treatment strategies, possibly in conjunction with current treatments.

However, very few studies have examined the molecular characteristics of residual cancer cells surviving chemotherapy. In a small study with 24 breast cancer patients receiving docetaxel alone as neoadjuvant chemotherapy, of whom 18 (75%) had ER-positive tumors, a gene expression pattern was identified in residual tumors, that included genes involved in cell cycle arrest at G2M and survival pathways involving the PI3K/mTor axis (Chang JC et al. J. Clin. Oncology 23: 1169-1177, 2005). More recently, studies reported an enrichment in breast cancer residual disease in cells with cancer stem cell (CSC) markers, such as cells with a CD44+/CD24-/low antigen profile or with mesenchymal features (Li X. et al. JNCI 100: 672-679, 2008), while other studies failed to confirm these observations (Aulman S. et al. Human Pathol. 41: 574-581, 2010). No obvious treatment strategy could be derived based on these observations.

Thus, the mechanisms leading to persistence of viable residual cancer cells, and later cancer recurrence, in patients whose tumor initially responds to chemotherapy are still largely unknown. Accordingly, there is currently no available biomarker able to predict if a breast cancer patient will respond to a given chemotherapy regimen, or to estimate the probability of relapse of a breast cancer patient harboring residual disease following neoadjuvant chemotherapy. The present invention discloses an original set of tumor biomarkers linked to the activation of the interferon (IFN)/Janus-activated kinase (Jak)/ signal transducer and activator of transcription (Stat) pathway in tumors exposed to chemotherapy *in vivo.* Their analysis in tumor tissue provides response predictive markers and novel therapeutic targets, which allows implementing specific adjuvant treatment strategies with improved antitumor efficacy.

The IFN/Stat signaling pathway and cancer biology. Members of the Stat family of transcription factors regulate the expression of a variety of genes involved in proliferation, differentiation, survival, and apoptosis (Levy DE, Darnell JE. Nat Rev Mol Cell Biol 2002; 3:651- 62). There are seven family members known to date, which are nuclear and cytoplasmic in location and provide a direct link between signals generated at cell surface receptors and regulation of gene expression in the nucleus. Many cytokines, growth factors, and hormones can lead to simultaneous activation of two or more Stat factors; however, targeted deletion of specific members has revealed cell type-specific roles with, for example, Stat1 being identified as the major effector of IFN-γ signaling (Ihle JA. Curr Opin Cell Biol 2001; 13:211-7).

The molecular events and signaling pathways that lie downstream of activated Stats have been largely determined from studies relevant to development and immune responses; however, recent years have seen the emergence of a role for select Stat family members in cancer (Yu H, Jove R. Nat Rev Cancer 2004; 4:97-105). Normally, Stat activation is a transient and tightly regulated process. However, in cancer, transient regulation is often replaced by constitutive activation.

Constitutive activation of Stat3 and Stat5 has been observed in a variety of tumor types including solid tumors of the breast, prostate, head and neck, as well as many leukemias and lymphomas. Their role in cell growth and survival is underpinned by their diverse gene targets, which include genes encoding inhibitors of apoptosis, such as Bcl-2 family members, proto-oncogenes such as c-Myc, and proliferative markers such as Pim-1. Furthermore, many reports describe how blocking constitutively activated Stat3 or Stat5 leads to apoptotic cell death in tumor cells (Yu H, Jove R. Nat Rev Cancer 2004; 4:97-105).

In contrast, loss of Stat1 protein expression has been observed in cancer (Yu H, Jove R. Nat Rev Cancer 2004; 4:97-105). The ability of IFN-γ to inhibit the growth of cells in culture is dependent on transcriptionally active Stat1 (Bromberg JF et al., Proc Natl Acad Sci USA 1996; 93:7673-8). This phenotype is reflected in the spectrum of its regulated target genes, including proteins involved in death receptor signaling (Fas) and those involved in cell cycle arrest (p21WAF1) (Ramana CV, Gil MP, Schreiber RD, Stark GR. Trends Immunol 2002; 2:96-101). Down-regulation of STAT1 has been observed in several tumor types. Therefore, Stat1 has properties of a tumor suppressor protein and not surprisingly has been suggested to antagonize the activities of Stat3 and 5 (Yu H, Jove R. Nat Rev Cancer 2004; 4:97-105).

Stat1 and cancer therapy. Cell death through apoptosis, senescence and mitotic catastrophe triggered by chemotherapy are key events in determining tumor growth and survival. Activation of Stat1 is generally considered as a pro-apoptotic event. For example, it was reported that doxorubicin potentiates Stat1 activation in response to IFN-γ in vitro, as this combination results in enhanced apoptosis in the MDA-MB435 human breast cancer cell line in a p53-independent manner (Thomas M et al. Cancer Res. 64: 8357-8364, 2004). These data show how Stat1 activation can be the basis of synergistic cell death observed in cells treated with both IFN-γ and doxorubicin. Cellular senescence represents a universal growth arrest program, which can be triggered by diverse stimuli including anticancer drugs. Recently, it was shown that drugs capable of inducing premature senescence in normal and cancer cells, such as 5-bromo-20-deoxyuridine (BrdU), distamycin A (DMA), aphidicolin and hydroxyurea, persistently activate STAT1 signaling and expression of interferon-stimulated genes (ISGs), such as MX1, OAS, ISG15, STAT1, PML, IRF1 and IRF7, in several human cancer cell lines (Novakova Z et al. Oncogene 29:273-284, 2010). Jak1/Stat-activating ligands, interleukin 10 (IL10), IL20, IL24, IFN-γ, IFN-β and IL6, were also expressed by senescent cells, supporting autocrine/paracrine activation of Jak1/Stat. Furthermore, cytokine genes, including pro-inflammatory IL1, tumor necrosis factor and transforming growth factor families, were highly expressed. Such cytokine production has been described in many cases of senescence and was called senescence-associated secretory phenotype (SASP).

Besides a large body of evidence suggesting that Stat1 plays a role in tumor suppression and drug-induced apoptosis or senescence, several studies have, on the other hand, implicated Stat1 in drug resistance and tumor progression.

A correlation was reported between high Stat1 expression and resistance to platinum-based chemotherapeutics in a panel of human ovarian carcinoma cell lines (Roberts D et al. B. J. Cancer 92: 1149-1158, 2005), or resistance to doxorubicin and radiation in an in vitro-selected doxorubicin-resistant clone of a human myeloma cell line (Fryknas M et al. Int J Cancer 120: 189-195, 2006). Persistent activation of the IFN/Stat1 pathway was found to be involved in acquisition of resistance to irradiation and IFN-γ in a human head and neck carcinoma cell line selected by repeated rounds of in vivo treatment with ionizing radiation in a xenograft model (Khodarev N et al. PNAS 101: 1714-1719, 2004). Stat1 pathway activation was manifested as overexpression of 52 genes, of whom 19 were known components of the IFN inducible pathway, including Stat1 itself. The results suggested that radio-resistance acquired during radiotherapy treatment, which may account for some treatment failures, is associated with up-regulation of the IFN-related STAT1 signaling pathway. These results have however not been confirmed in the clinical setting.

The gene expression profile associated with resistance to radiation described in the above study, termed IFN-related DNA damage signature (IRDS), was examined in series of human tumors through unsupervised clustering analysis of microarray datasets. This study revealed that IRDS(+) and IRDS(-) states exist among common human tumors including breast, lung, prostate and glioblastomas (Weichselbaum R PNAS 105: 18490-18495, 2008). Based on this and other studies from the same laboratory, a clinical value for Stat1 in cancer diagnostics has been reported in the patent application US087964, where a seven-gene pair classifier extracted from the IRDS is presented as a predictor for the efficacy of adjuvant chemotherapy and for loco-regional control after radiation of breast and other cancers. The IRDS signature is also presented as useful for assessing risk of local-regional failure, survival and metastasis in breast cancer patients.

In another study however, the presence of activated Stat1 in a panel of breast cancers was shown to be a significant indicator of good prognosis, even after adjusting for known prognostic variables (lymph node status, stage of disease, estrogen receptor status, and cathepsin D) (Widschwendter A. et al., Clin Cancer Res 2002; 8:3065-74.).

In conclusion, numerous studies indicate opposite functions in tumor suppression and response to chemotherapy for the different members of the Stat family. Notably, the relationship between activation of Stat1 signaling and cancer chemotherapy is presently unclear and no methods have been yet developed to exploit this pathway therapeutically.

### 3. SUMMARY OF THE INVENTION

The present invention relates to methods and compositions that utilize a particular panel of biomarkers and their expression signatures, wherein the expression signatures predict responsiveness, or lack thereof of human tumor cells, to chemotherapy treatment. The invention is based on the identification of a specific set of biomarkers that are differentially expressed in chemotherapy-treated tumors and which are useful in predicting the likelihood of a therapeutic response, including tumor regression, residual disease persistence and subsequent tumor recurrence in cancer patients receiving chemotherapy. In chemo-sensitive tumors, a large subset of the genes that are over-expressed in residual tumor cells from treated tumors compared to pretreatment tumor cells correspond to a gene cluster regulated by the IFN/Stat signaling pathway. Accordingly, the present invention provides methods of early prediction of tumor response in patients subjected to chemotherapy. The invention is directed to a method of predicting tumor response and patient relapse in a patient subjected to chemotherapy comprising (i) measuring biomarker expression in a sample of cancer cells from a subject and (iii) predicting the likelihood of a response to chemotherapy based on the pattern of biomarker expression. The identified differential biomarker expression pattern, including those biomarkers regulated by the IFN/Stat signaling pathway, between chemo-sensitive and chemo-resistant tumors provides for early prediction of tumor responsiveness, as well as tumor recurrence, in cancer subjects. The methods of the invention rely on measurement of the expression level of one or more predictive RNA transcripts, and/or of their expression products, including their post-translational modification, in a cancer cell obtained from the patient. The measures obtained are normalized against the expression level of all or a reference set of RNA transcripts or their expression products, wherein a predictive RNA transcript or its product is the transcript or product of a gene selected from the group consisting of the genes of Table 3, the gene exons of Table 4 and/or the micro-RNAs of Table 5.

In a specific embodiment of the invention, the definition of biomarker covers post-translational modifications of gene products related to the activation of the IFN/Stat pathway.

Another object of the present invention is to provide methods for the selection of an appropriate cancer treatment and predicting the outcome of the same. The identified link between high biomarker expression, such as IFN/Stat marker expression, and responsiveness to DNA-damaging drugs will form the basis for a decision to apply a specific regimen for treatment of the subject. Thus, in this aspect, the present invention provides a method for treatment of a cancer in a subject in need thereof, comprising the steps of: a) measuring the amount and intensity of biomarker expression present in a tumor sample derived from a subject, and determining a sample value corresponding to said measurements; b) comparing the sample value obtained in step a) with a reference value, and depending on the sample/reference ratio obtained (greater than, equal to, or less than 1), c) treating said subject with the appropriate treatment regimen identified for each of the three classes.

In a specific embodiment of the invention, the biomarkers may be those biomarkers regulated by the IFN/Stat signaling pathway, as indicated in Table 3. In another embodiment of the invention, the biomarkers include any combination of part or all of the genes of Table 3, of the exons of Table 4 and/or of the microRNAs of Table 5. Marker-positive tumors are predicted to be sensitive to chemotherapy, while marker-negative tumors are predicted to be resistant to chemotherapy, and patients with marker-negative tumors can be spared the adverse side effects of a treatment that is unlikely to be beneficial. When available, alternative treatment can be administered accordingly. Conversely, marker-positive tumors are likely to be responsive to chemotherapy. Patients with marker-positive tumors can benefit from the addition of a treatment that targets the oncogenic mechanisms activated in the marker-positive tumors as detailed further below. Tailoring treatment to the patient based on marker status will result in both cost savings and toxicity sparing by eliminating administration of ineffective treatments, and in improved clinical outcome by implementing specific adjuvant treatment based on marker expression.

In another embodiment of the invention, a xenograft model system is provided for identifying a biomarker expression signature that is correlated with drug response and clinical outcome. The method includes a) developing a xenograft model showing response to therapy followed by tumor relapse, b) identifying genes differentially expressed between the residual and pre-treatment tumor wherein the differentially expressed genes, i.e., biomarkers, form a drug response expression signature, c) determining the drug response expression signature status of tumors from a population of humans, and d) correlating the resistance expression signature status with drug response and clinical outcome.

In a specific embodiment of the invention, the xenograft model is obtained from direct grafting of a fresh human tumor sample onto immunodeficient mice. In another embodiment of the invention, the tumor xenograft tissue used for analysis is processed by laser-capture microdissection of frozen section, in order to isolate tumor cells from surrounding murine stromal components.

The invention also provides kits for measuring the level of biomarker expression in a sample. The kits may include one or more reagents corresponding to the biomarkers described herein, e.g., antibodies that specifically bind the biomarkers, recombinant proteins that bind biomarker specific antibodies, nucleic acid probes or primers that hybridize to the biomarkers, etc. In some embodiments, the kits may include a plurality of reagents, e.g., on an array, corresponding to the biomarkers described herein. The kits may include detection reagents, e.g., reagents that are detectably labeled. The kits may include written instructions for use of the kit in predicting the likelihood of a therapeutic response in a cancer patient being treated with a chemotherapeutic reagent, and may include other reagents and information such as control or reference standards, wash solutions, analysis software, etc.

More particularly, the invention relates to a composition comprising (i) a means for detecting one or more biomarkers which are expressed by drug-sensitive human tumor cells during a chemotherapeutic drug treatment or by drug-resistant tumor residual cells found after treatment of a drug-sensitive tumor by at least one chemotherapeutic drug, wherein said biomarker is selected from those differentially expressed biomarkers of Table 3, 4 and/or 5 and (ii) a sample derived from a human tumor cell.

In a specific embodiment of the invention, the chemotherapeutic drug is a chemotherapeutic agent. Advantageously, the biomarker:
- is regulated by the IFN/STAT signaling pathway, or
- is one or more of the following biomarkers the expression of which is predictive of tumor sensitivity to a drug used during chemotherapy: DTX3L, CCL5, IFIT1, IFITM1, IRF9, IFI6, IFI44, IFI44L, OAS1, OAS2, LAMP3, MX1, PARP9, PARP12, PARP14, SAMD9, SAMD9L, BST2, DDX60, CLDN1,STAT1, STAT2, UBE2L6, ZNFX1, or
- is a polypeptide, peptide or polynucleotide or nucleotide sequences the expression or post-translational modification of which is predictive of tumor sensitivity to a drug used during chemotherapy, or
- is specific for an early cell response corresponding to a tumor sensitivity to an anti tumoral treatment comprising chemotherapeutic drugs, said biomarker being one or more of the following: DTX3L, CCL5, IFIT1, IFITM1, IRF9, IFI6, IFI44, IFI44L, OAS1, OAS2, LAMP3, MX1, PARP9, PARP12, PARP14, SAMD9, SAMD9L, BST2, DDX60, CLDN1,STAT1, STAT2, UBE2L6, ZNFX1, or
- predicts a tumor's sensitivity to an anti-tumoral treatment comprising chemotherapeutic drugs, said biomarker being one or more of the following: DTX3L, CCL5, IFIT1, IFITM1, IRF9, IFI6, IFI44, IFI44L, OAS1, OAS2, LAMP3, MX1, PARP9, PARP12, PARP14, SAMD9, SAMD9L, BST2, DDX60, CLDN1,STAT1, STAT2, UBE2L6, ZNFX1, or
- predicts sensitivity of a breast cancer tumor to a primary antitumoral therapy with optionally an added adjuvant, said biomarker being one or more of the following: DTX3L, CCL5, IFIT1, IFITM1, IRF9, IFI6, IFI44, IFI44L, OAS1, OAS2, LAMP3, MX1, PARP9, PARP12, PARP14, SAMD9, SAMD9L, BST2, DDX60, CLDN1,STAT1, STAT2, UBE2L6, ZNFX1, or
- is predictive of a tumor cell's sensitivity to anti-tumoral therapy, said biomarker being a modified or mutated exons of Table 4 or a micro-RNA of table 5.

The invention also relates to a method of predicting tumor response in a patient subjected to chemotherapy comprising (i) measuring the amount of expression in a sample of cancer cells from a subject of a differentially expressed biomarker wherein said biomarker is selected from those differentially expressed biomarkers of Table 3, 4 and/or 5 and (iii) predicting the likelihood of a response to chemotherapy based on the expression of the biomarker. Advantageously, the biomarker:
- is regulated by the IFN/STAT signaling pathway, or
- is one or more of the following biomarkers the expression of which is predictive of tumor sensitivity to a drug used during chemotherapy: DTX3L, CCL5, IFIT1, IFITM1, IRF9, IFI6, IFI44, IFI44L, OAS1, OAS2, LAMP3, MX1, PARP9, PARP12, PARP14, SAMD9, SAMD9L, BST2, DDX60, CLDN1,STAT1, STAT2, UBE2L6, ZNFX1.

The invention also relates to a method for analyzing differential expression of biomarker, comprising the step of: measuring the amount of biomarker expression present in a tumor sample derived from a subject, and determining a sample value corresponding to said amount wherein said biomarker is selected from those differentially expressed biomarkers of Table 3, 4 and/or 5, (ii) comparing the sample value obtained in step (i) with a reference value. Advantageously, the biomarker:
- is regulated by the IFN/STAT signaling pathway, or
- is one or more of the following biomarkers the expression of which is predictive of tumor sensitivity to a drug used during chemotherapy: DTX3L, CCL5, IFIT1, IFITM1, IRF9, IFI6, IFI44, IFI44L, OAS1, OAS2, LAMP3, MX1, PARP9, PARP12, PARP14, SAMD9, SAMD9L, BST2, DDX60, CLDN1,STAT1, STAT2, UBE2L6, ZNFX1.

This method can be included in a method for treatment of a cancer in a subject in need thereof, comprising the steps of: (i) measuring the amount of biomarker expression present in a tumor sample derived from a subject, and determining a sample value corresponding to said amount wherein said biomarker is selected from those differentially expressed biomarkers of Table 3, 4 and/or 5; (ii) comparing the sample value obtained in step (i) with a reference value, and depending on the sample/reference ratio obtained (greater than, equal to, or less than 1); (iii) treating said subject with a specific treatment regimen identified for each of the three classes. In a specific embodiment, treatment is a drug that is an inhibitor of at least one of the differentially expressed biomarkers of Table 3, 4 and/or 5. The treatment can be aimed at activating the IFN/Stat pathway in the cells of a chemotherapy-resistant tumor.

The invention further relates to a process for identification of an anti-tumoral molecule capable of inhibiting biomarker expression comprising the steps of (i) contacting a cell expressing at least one expressed exon corresponding to one of the biomarkers of Table 4 expressed by residual tumoral cell after treatment at high or lethal dose with a chemotherapeutic drug, with standard diluted concentrations of the anti-tumoral molecule of interest and (ii) measuring the inhibitor effect of the molecule of interest tested on the expressed exon; and (iii) optionally comparing the percentage of inhibition to a non-treated cell with such molecule.

The invention also concerns a process for identification of an anti-tumoral molecule comprising the step of contacting a candidate anti-tumoral molecule to be tested with at least one expressed biomarker wherein said biomarker is selected from those differentially expressed biomarkers of Table 3, 4 and/or 5, wherein the biomarker is specific for the human residual cells resistant to a lethal dose of a chemotherapeutic drug, said human resistant cells being capable to divide in an animal model, *in vivo.*

The invention provides a xenograft animal model comprising human xenograft cells which are resistant to chemotherapeutic drugs, said cells expressing at least one of the differentially expressed biomarkers of Table 3, 4, and/or 5. Advantageously, the biomarker:
- is regulated by the IFN/STAT signaling pathway, or
- is one or more of the following biomarkers the expression of which is predictive of tumor sensitivity to a drug used during chemotherapy: DTX3L, CCL5, IFIT1, IFITM1, IRF9, IFI6, IFI44, IFI44L, OAS1, OAS2, LAMP3, MX1, PARP9, PARP12, PARP14, SAMD9, SAMD9L, BST2, DDX60, CLDN1,STAT1, STAT2, UBE2L6, ZNFX1.

The invention also concerns the use of at least one of the differential expressed biomarkers of Table 3, 4, and/or 5 for the detection of residual tumoral cells after treatment of human breast, colon or lung cancer cells by a chemotherapeutic drug at high or lethal dose. Advantageously, the biomarker:
- is regulated by the IFN/STAT signaling pathway, or
- is one or more of the following biomarkers the expression of which is predictive of tumor sensitivity to a drug used during chemotherapy: DTX3L, CCL5, IFIT1, IFITM1, IRF9, IFI6, IFI44, IFI44L, OAS1, OAS2, LAMP3, MX1, PARP9, PARP12, PARP14, SAMD9, SAMD9L, BST2, DDX60, CLDN1,STAT1, STAT2, UBE2L6, ZNFX1.

The invention provides a process for detection *in vitro* of at least one of the differential expressed biomarkers of Table 3, 4, and/or 5 expressed by human tumor cells after treatment by at least one chemotherapeutic drug comprising contacting said human tumor cell with a reagent capable of detecting said biomarker. Advantageously, the reagent is a nucleic acid probe that selectively binds to a nucleic acid encoding said biomarker or an antibody molecule that binds selectively to the biomarker.

The invention also relates to the use of at least one of the differentially expressed biomarkers of Table 3, 4 and/or 5, as a therapeutic target for the adjuvant treatment associated optionally to the chemotherapy.

Finally, the invention provides a treatment of a patient affected by a breast, colon or lung cancer comprising administration of a chemotherapeutic drug in combination with a drug that is an inhibitor of at least one of the differentially expressed biomarkers of Table 3, 4 and/or 5. Preferably, the administration of said treatment follows the early detection of the biomarkers after administration of a chemotherapeutic drug or follows detection of the biomarkers in residual tumor cells surviving chemotherapeutic drug treatment of a breast cancer. More particularly, the invention provides a combination of a chemotherapeutic drug with a drug that is an inhibitor of at least one of the differentially expressed biomarkers of Table 3, 4 and/or 5 for use as a medicament, in particular for the treatment of a breast, colon or lung cancer.

In a specific embodiment, the chemotherapeutic drug of the treatment is composed of a combination of Adriamycine and Cyclophosphamide. Alternatively, one of the drugs used for chemotherapy is a genotoxic agent, such as but not limited to Cyclophosphamide, Etoposide, Ifosfamide, cis-platinum or Irinotecan.

In another specific embodiment, the inhibitor of the differentially expressed biomarker is a member of the PARP inhibitor group.

The present invention also relates to:
1. A composition comprising (i) a means for detecting one or more biomarkers which are expressed by drug-sensitive human tumor cells during a chemotherapeutic drug treatment or by drug-resistant tumor residual cells found after treatment of a drug-sensitive tumor by at least one chemotherapeutic drug, wherein said biomarker is selected from those differentially expressed biomarkers of Table 3, 4 and/or 5 and (ii) a sample derived from a human tumor cell.
2. The composition of 1., wherein the biomarker is one or more of the following biomarkers the expression of which is predictive of tumor sensitivity to a drug used during chemotherapy: DTX3L, CCL5, IFIT1, IFITM1, IRF9, IFI6, IFI44, IFI44L, OAS1, OAS2, LAMP3, MX1, PARP9, PARP12, PARP14, SAMD9, SAMD9L, BST2, DDX60, CLDN1, STAT1, STAT2, UBE2L6, ZNFX1.
3. The composition of 1. wherein the biomarker is a polypeptide, peptide or polynucleotide or nucleotide sequences the expression or post-translational modification of which is predictive of tumor sensitivity to a drug used during chemotherapy.
4. The composition of 1., wherein the biomarker is predictive of a tumor cell's sensitivity to anti-tumoral therapy, said biomarker being a modified or mutated exons of Table 4 or a micro-RNA of table 5.
5. A method of predicting tumor response in a patient subjected to chemotherapy comprising (i) measuring the amount of expression in a sample of cancer cells from a subject of a differentially expressed biomarker wherein said biomarker is selected from those differentially expressed biomarkers of Table 3, 4 and/or 5 and (iii) predicting the likelihood of a response to chemotherapy based on the expression of the biomarker.
6. The method of 5., wherein the biomarker is one or more of the following biomarkers the expression of which is predictive of tumor sensitivity to a drug used during chemotherapy: DTX3L, CCL5, IFIT1, IFITM1, IRF9, IFI6, IFI44, IFI44L, OAS1, OAS2, LAMP3, MX1, PARP9, PARP12, PARP14, SAMD9, SAMD9L, BST2, DDX60, CLDN1, STAT1, STAT2, UBE2L6, ZNFX1.
7. A method for analyzing differential expression of biomarker comprising the steps of: (i) measuring the amount of biomarker expression present in a tumor sample derived from a subject, and determining a sample value corresponding to said amount wherein said biomarker is selected from those differentially expressed biomarkers of Table 3, 4 and/or 5, (ii) comparing the sample value obtained in step (i) with a reference value.
8. The method of 7., wherein the biomarker is one or more of the following biomarkers the expression of which is predictive of tumor sensitivity to a drug used during chemotherapy: DTX3L, CCL5, IFIT1, IFITM1, IRF9, IFI6, IFI44, IFI44L, OAS1, OAS2, LAMP3, MX1, PARP9, PARP12, PARP14, SAMD9, SAMD9L, BST2, DDX60, CLDN1, STAT1, STAT2, UBE2L6, ZNFX1.
9. Use of at least one of the differential expressed biomarkers of Table 3, 4, and/or 5 for the detection of residual tumoral cells after treatment of human breast, colon or lung cancer cells by a chemotherapeutic drug at high or lethal dose.
10. The use of 9., wherein the biomarker is one or more of the following biomarkers the expression of which is predictive of tumor sensitivity to a drug used during chemotherapy: DTX3L, CCL5, IFIT1, IFITM1, IRF9, IFI6, IFI44, IFI44L, OAS1, OAS2, LAMP3, MX1, PARP9, PARP12, PARP14, SAMD9, SAMD9L, BST2, DDX60, CLDN1, STAT1, STAT2, UBE2L6, ZNFX1.
11. A process for detection *in vitro* of at least one of the differential expressed biomarkers of Table 3, 4, and/or 5 expressed by human tumor cells after treatment by at least one chemotherapeutic drug comprising contacting said human tumor cell with a reagent capable of detecting said biomarker.
12. The process of 11., wherein the reagent is a nucleic acid probe that selectively binds to a nucleic acid encoding said biomarker.
13. The process of 11., wherein the reagent is an antibody molecule that binds selectively to the biomarker.
14. Use of at least one of the differentially expressed biomarkers of Table 3, 4 and/or 5, as a therapeutic target for the adjuvant treatment associated optionally to the chemotherapy.
15. Combination of a chemotherapeutic drug with a drug that is an inhibitor of at least one of the differentially expressed biomarkers of Table 3, 4 and/or 5 for use as a medicament.
16. Combination of 15., for the treatment of a breast, colon or lung cancer.

### 4. BRIEF DESCRIPTION OF THE FIGURES

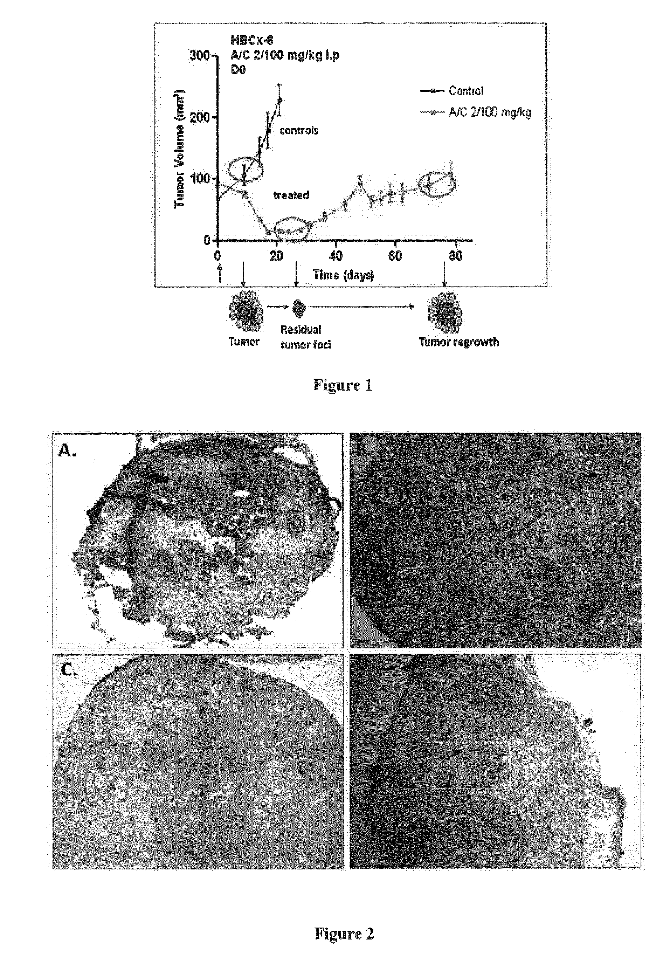

**Figure 1****. Tumor recurrence following A/C combination therapy on HBCx-6.** A/C was administrated once by intraperitoneal route at 2/100 mg/kg at D0. Complete tumor regressions were observed in 96% of treated mice 19 days after treatment, followed by tumor recurrence for all tumors. Tumor samples were collected at different steps as follows: 5 "Controls" (= untreated tumors), 5 "Nodules" (= residual tumor cells), 5 "Regrowths" (= tumor relapse).
**Figure 2****. Histology and Microdissection of Residual Tumors after Chemotherapy (x200).** Residual tumors after cresyl violet staining of frozen section. (A) HBCx-6; (B) HBCx-8; (C) HBCx-10: (D) HBCx-17: Foci of tumor cells (circled in red) are surrounded by a fine murine stroma composed of fibroblasts and infiltrating inflammatory cells, along with necrotic areas.

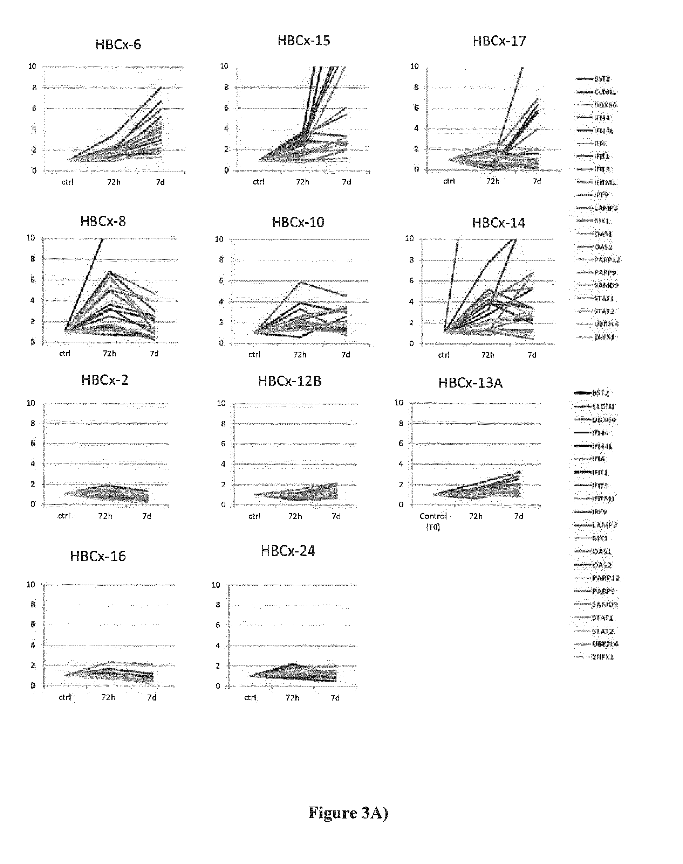

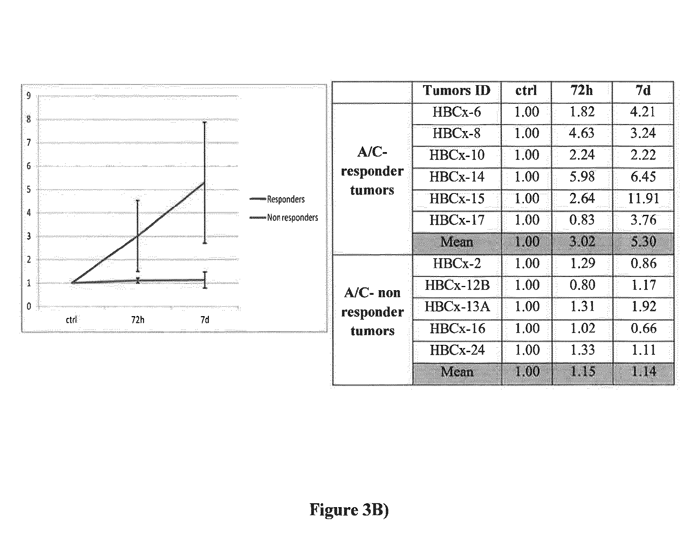

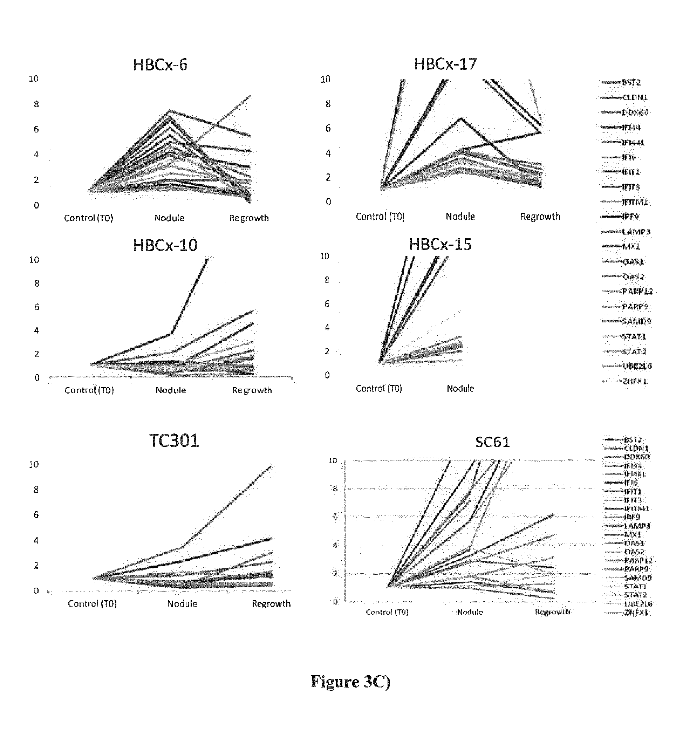

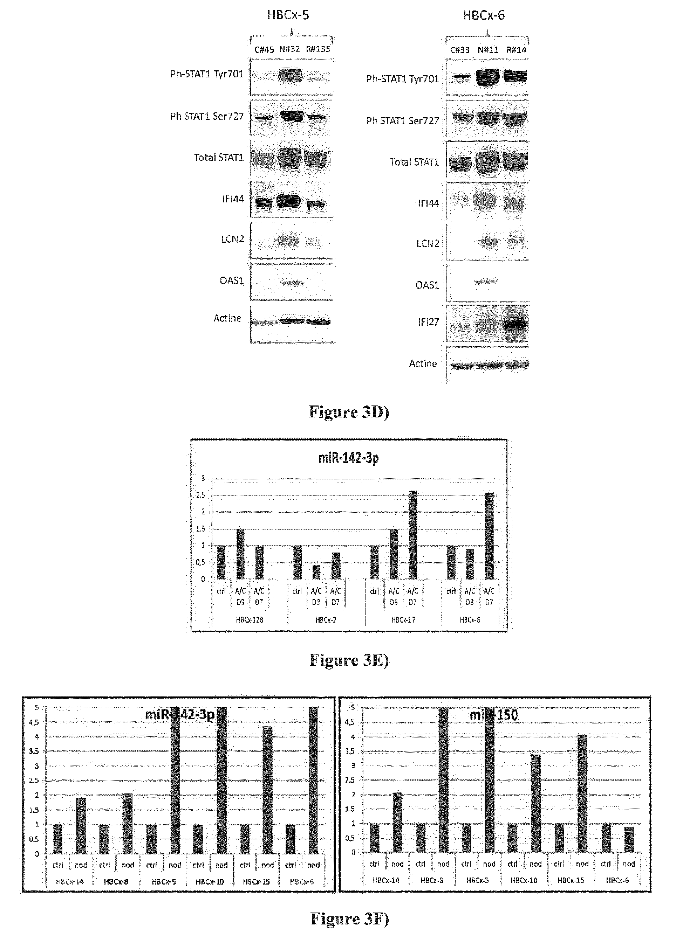

**Figure 3****. Quantitative PCR (qPCR) and western blotting analyses of selected genes, microRNAs and proteins in tumors before and after chemotherapy.** 3A) Expression profile of a 21-gene signature in untreated control samples (ctrl), and at 72 hours (72h) and 7 days (7d) post- A/C treatment in 6 responder (HBCx-6, HBCx-8 HBCx-10, HBCx-14, HBCx-15, HBCx-17) and 5 non-responder (HBCx-2, HBCx-12B, HBCx-13A, HBCx-16, HBCx-24) breast cancer xenograft models. 3B) Mean relative expression values of 10 genes of the 21-gene signature with best variation coefficient (CV) in untreated control samples (ctrl), and at 72 hours (72h) and 7 days (7d) post- A/C treatment in 6 responder (HBCx-6, HBCx-8 HBCx-10, HBCx-14, HBCx-15, HBCx-17) and 5 non-responder (HBCx-2, HBCx-12B, HBCx-13A, HBCx-16, HBCx-24) breast cancer xenograft models (vertical bars: standard error). 3C) Expression profile of a 21-gene signature at nodule and regrowth phase in various responder tumors: 4 breast cancer xenograft models treated with A/C (HBCx-6, HBCx-10, HBCx-15 and HBCx-17); one small-cell lung cancer xenograft model (SC61) treated with etoposide/ifosfamide/cisplatin combination; one colorectal cancer xenograft model (TC301) treated with irinotecan. 3D) Expression at the nodule and regrowth stage of 5 proteins encoded by 5 genes of the IFN/Stat signature (Stat1, Ifi27, If44, Lcn2, Oasl) and of phosphorylated Stat1 in 2 breast cancer xenograft models responding to A/C treatment (HBCx-5 and HBCx-6). 3E) Expression of miR-142-3p in untreated control samples (ctrl), and at day 3 (D3) and 7 days (D7) post-A/C treatment in two responder (HBCx-6 and HBCx-17) and two non-responder (HBCx-2 and HBCx-12B) breast cancer xenograft models. 3F) Expression of miR-142-3p and miR-150 at the nodule and regrowth stage following A/C treatment in 6 responder breast cancer xenograft models (HBCx-5, HBCx-6, HBCx-8, HBCx-10, HBCx-14, HBCx-15).

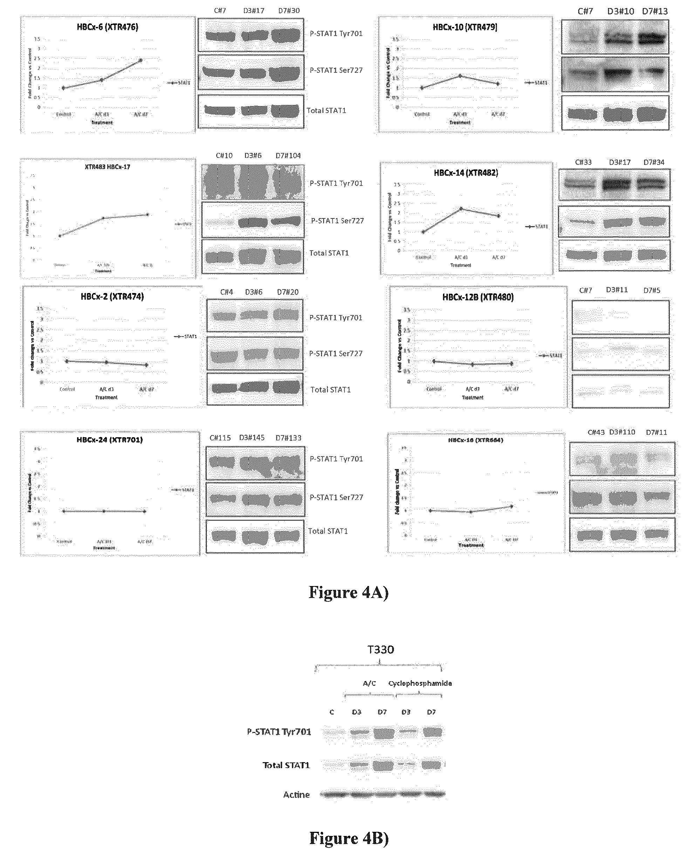

**Figure 4****. Stat1 expression in tumorgrafts before and after chemotherapy. 4A)** Parallel qPCR and western blotting analyses of Stat1 expression in the same tumor specimens before and after chemotherapy. 4A) Expression of Stat1 in untreated control samples (C), and at 3 days (D3) and 7 days (D7) post- A/C treatment in 4 responder (HBCx-6, HBCx-10 HBCx-14, HBCx-17) and 4 non-responder (HBCx-2, HBCx-12B, HBCx-16, HBCx-24) breast cancer xenograft models. The graphs represent Stat1 gene expression levels determined by qPCR. Western blots show expression levels of either total or Tyr701 and Ser727 phosphorylated Stat1 protein isoforms. **4B)** Western blotting analysis of Stat1 expression before and after chemotherapy. The same increase in Stat1 protein amount and phosphorylation level is observed in T330 breast cancer xenograft treated with either cyclophosphamide alone or in combination with adriamycin.

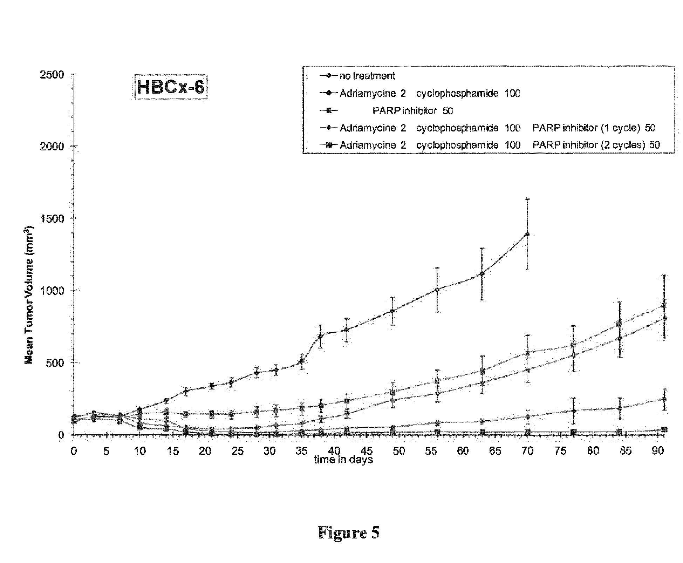

**Figure 5****. Combination of A/C with PARP inhibitor treatments induces sustained tumor regression and prevents tumor recurrence in the human breast tumor xenograft HBCx-6.** A/C was administered i.p once at 2/100 mg/kg. PARP1 inhibitor was administered at 50 mg/kg i.p qdx10. All treatments started concurrently at D0. In addition, an additional cohort was sequentially treated with a second cycle of PARP inhibitor at 50 mg/kg (qdx5) during the nodule phase (e.g. between D20 and D30 for most mice). Data are expressed as mean tumor volume (mm3).

### 5. DETAILED DESCRIPTION OF THE INVENTION

The present invention provides a specific set of biomarkers that are differentially expressed in chemotherapy-treated tumors. Such biomarkers, as described in detail below, may be used in methods designed to predict the likelihood of a therapeutic response, including tumor regression, residual disease persistence and subsequent recurrence in cancer patients receiving chemotherapy.

### 5.1. BIOMARKERS OF THE INVENTION

To study the mechanisms underlying tumor regression, residual cancer disease persistence and tumor recurrence following chemotherapy, a model system was developed based on human patient tumor-derived murine xenografts (eg. breast, colon, lung, or brain tumor type). Several tumor models highly responsive to chemotherapy underwent complete macroscopic tumor regression, followed by tumor recurrence after a period of several weeks. Therefore, while chemotherapy was able to kill the majority of tumor cells, a small population of tumor cells survived chemotherapy and formed the basis of subsequent tumor relapse.

As described in detail below, tumor cells from untreated tumors and post-chemotherapy residual tumor nodules were isolated by laser-capture microdissection of frozen sections from tumor tissue harvested at the tumor graft site. RNA was extracted and used to study global gene expression regulation at gene and exon level as well as microRNA expression.

Comparison of gene expression levels between pre-treatment and post-chemotherapy residual tumor cells identified a gene expression signature composed of over-expressed and under-expressed genes, common to several tumor models tested (Table 3). A large subset of the genes that were over-expressed in residual tumor cells compared to pre-treatment tumor cells corresponded to a gene cluster regulated by the IFN/Stat signaling pathway (Table 3). Gene sequences corresponding to each of the listed genes of Table 3, the exons of Table 4 and the micro RNAs of Table 5 are publicly available, for example in Genbank.

An additional set of tumor biomarkers was provided by analyzing RNA transcript expression at the exon level, which led to the identification of RNA transcript isoforms differentially expressed between untreated and residual tumor cells (Table 4). These transcript isoforms reflect alternative splicing events such as: exon skipping (simple cassette exon, multiple cassette exons, mutually exclusive exons), alternative 5'/3' splice sites (e.g. alternative exon-intron and intron-exon sites respectively) and intron retention. Such alternative splicing events may also correspond to alternative promoters and/or terminal exons usage. Most importantly, these alternative RNA splicing isoforms may give rise to the translation of corresponding protein isoforms with altered functionalities.

Another set of tumor biomarkers was provided by microRNA expression profiling, which led to the identification of microRNAs differentially expressed between untreated and residual tumor cells (Table 5). MicroRNAs regulate the stability of gene transcripts and their translation into proteins. They constitute an interesting class of biomarkers that can be easily measured in blood and tissues, and also provide potential therapeutic targets.

qPCR assays were developed for 21 gene transcripts of the identified expression signature. The 21-gene list is detailed in Table 1, and they are marked in bold in Table 3, where differential gene expression in early post-treatment (24h to 21 days) and residual tumors versus untreated tumors is listed. These genes are: IFI44L, LAMP3, OAS2, PARP9, IFIT1, STAT2, OAS1, IRF9, UBE2L6, BST2, MX1, IFIT3, IFI44, DDX60, IFI6, STAT1, SAMD9, ZNFX1, IFITM1, PARP12, CLDN1. Time-course experiments were performed to analyze their variation in several tumor models upon treatment with chemotherapies. Results showed that increased expression of several genes could be detected between 24h and 21 days after treatment in tumors that responded to chemotherapy (eg. those forming residual nodules following drug-induced tumor regression). On the other hand, no increased gene expression was detected in tumors not responding to chemotherapy (Fig.3A-B). Table 6 (a-b) depicts the expression profile of IFN/Stat-related gene expression in tumor xenograft models.

Several microRNAs were also found differentially expressed between residual tumor cells from tumors treated with chemotherapy and untreated tumors (Table 5). Expression of two of these micro-RNAs: miR-142-3p and miR-150 was measured by qPCR, which showed an increased expression of these two microRNAs in early post-treatment and residual tumors versus untreated tumors only in tumors responding to chemotherapy (Fig. 3E and F). No increased gene/microRNA expression was detected in tumors not responding to chemotherapy.

Western blotting assays with total lysate from the same tumor specimens analyzed by qPCR were performed using antibodies directed against 5 proteins encoded by the genes listed in Table 3, and two phosphorylated forms of Stat1. These proteins are: Stat1, Ifi27, If44, Lcn2 and Oas1. Time-course experiments were performed to analyze their variation in several tumor models upon treatment with chemotherapies. Results showed that increased expression of several proteins and increased phosphorylation of Stat1 could be detected between 24h and 21 days after treatment in tumors that responded to chemotherapy (eg. those forming residual nodules following drug-induced tumor regression). On the other hand, no increase in protein expression or Stat1 phosphorylation level was detected in tumors not responding to chemotherapy (Fig.3D and 4)

Accordingly, the present invention provides methods of early prediction of tumor response in cancer patients subjected to chemotherapy. The methods of the invention rely on measurement of the expression level of one or more predictive RNA transcripts, and/or of their expression products, including their post-translational modification, in a cancer cell obtained from a patient subjected to chemotherapy. The measurements obtained are normalized against the expression level of all or a reference set of RNA transcripts or their expression products, wherein a predictive RNA transcript or its product is the transcript or the product of a gene belonging to the group of genes and exons listed in Table 3 and Table 4 and/or the microRNAs of Table 5.

Moreover, our observation of increased phosphorylation of Stat1, a typical marker of IFN/Stat signaling pathway activation, allows the extension of biomarker definition to all post-translational modifications related to the activation of the IFN/Stat pathway. Evaluation if a biomarker belongs to said pathway is within the ability of one skilled in the art.

The observation that the IFN/Stat signaling pathway is activated following chemotherapy specifically in responsive tumors points to a contribution of tumor suppressor components of this pathway in the antitumor effect of chemotherapy. On the other hand, several components of this pathway have a potential protective role that could contribute to the selection and survival of residual cancer cells. To test whether the genes or pathways whose expression is increased in residual cancer cells following chemotherapy could contribute to protection of these cells from death or to increase their DNA repair capacity, experimental validations were determined in tumor xenografts that combined conventional chemotherapy and selected drugs to inhibit protective mechanisms that were presumably activated in drug-resistant residual cells. In a specific embodiment of the invention, Parp family members, several of which belong to the IFN/Stat-regulated genes whose expression is increased following A/C treatment and in residual tumor cells, were targeted using the tumor xenograft model. Results in the HBCx-6 breast cancer xenograft model showed that the combination of A/C with a PARP inhibitor had improved antitumor efficacy compared to A/C treatment alone (Fig.5). Combined treatment with A/C and one early cycle of the PARP inhibitor (from day 0 to day 9) resulted in delayed tumor relapse, while combination of A/C with 2 cycles of PARP inhibitor (early from day 0 to day 9 and at the nodule phase from day 20 to day 30) resulted in complete suppression of tumor relapse. These data identify Parp function as a relevant therapeutic target in A/C-treated tumors, whose inhibition is able to prevent or delay tumor relapse when combined with conventional chemotherapy.

Accordingly, the present invention provides methods for identifying an expression signature biomarker that is correlated with drug response and clinical outcome. The method includes a) developing a xenograft model showing response to therapy followed by tumor relapse, b) identifying genes differentially expressed between the residual and pre-treatment tumor wherein the differentially expressed genes forms a drug response expression signature, c) determining the drug response expression signature status of tumors from a patient population, and d) correlating the resistance expression signature status with drug response and clinical outcome.

The present invention provides compositions comprising biomarkers, e.g., nucleic acid molecules and expression products thereof, or means for detecting said biomarkers, wherein the biomarkers are found to be differentially expressed tumor cells that are responsive to chemotherapy as compared to tumor cells that are non-responsive to chemotherapy.

As used herein a "biomarker" is a molecular indicator of a specific biological property and as used herein is a nucleic acid molecule (e.g., a gene or gene fragment), an expression product thereof (e.g., a RNA, microRNA, a polypeptide or peptide fragment or variant thereof) or any detectable modification of said products (phosphorylation, acetylation, glycosylation etc.) whose differential detection (presence, absence, over-expression or under-expression relative to a reference) within a cell or tissue indicates the likelihood of a therapeutic response to chemotherapy. An "expression product" as used herein is a transcribed sense or antisense RNA molecule (e.g., an mRNA), or a translated polypeptide corresponding to or derived from a polynucleotide sequence. A "panel" of biomarkers is a selection of two or more combinations of biomarkers.

Biomarkers for characterizing, or subtyping, the different types of tumors, according to the invention, include those listed in Tables 3-5. Such markers include genes that are found to be regulated by the IFN/STAT signaling pathway. One or more of these biomarkers, or up to all of the biomarkers, may be used together in any combination in the methods according to the invention.

As indicated above, nucleic acid sequences encoding the biomarkers of the invention, are publicly available (for example, accessible in GenBank), known to those of skill in the art, and incorporated herein in their entirety. As described in detail below, such nucleic acid sequences may be used to design probes or primers for use in assays for measuring the levels of biomarker expression in a cancer cell.

Biomarkers according to the invention include substantially identical homologues and variants of the nucleic acid molecules and expression products thereof described herein, for example, a molecule that includes nucleotide sequences encoding polypeptides functionally equivalent to the biomarkers of the invention, e.g, sequences having one or more nucleotide substitutions, additions, or deletions, such as allelic variants or splice variants or species variants or molecules differing from the nucleic acid molecules and polypeptides referred to in the Tables herein due to the degeneracy of the genetic code.

Other nucleic acids for use in the practice of the invention include those that have sufficient homology to those described herein to detect expression by use of hybridization techniques. Such polynucleotides preferably have about or 95%, about or 96%, about or 97%, about or 98%, or about or 99% identity with the biomarker sequences as described herein. The other polynucleotides for use in the practice of the invention may also be described on the basis of the ability to hybridize to polynucleotides of the invention under stringent conditions of about 30% v/v to about 50% formamide and from about 0.01M to about 0.15M salt for hybridization and from about 0.01M to about 0.15M salt for wash conditions at about 55 to about 65°C, or higher, or conditions equivalent thereto.

While individual biomarkers are useful diagnostics, the combination of biomarkers as proposed herein, enables accurate determination of the likelihood of responding to chemotherapy.

### 5.2. BIOMARKER DETECTION

Determining the expression levels of the biomarkers described herein enables a medical practitioner to determine the appropriate course of action for a subject (e.g, chemotherapy, surgery, no action, etc.) based on the observed expression signature. Detection of the biomarkers described herein may also help determine the prognosis for a given cancer, subtyping of the cancer, evaluation of the efficacy of a therapy for cancer, monitoring a cancer therapy in a subject, or detecting relapse of cancer in a subject who has undergone therapy for cancer and is in remission. In alternative aspects, the biomarkers and reagents prepared using the biomarkers may be used to identify novel cancer therapeutics.

Expression levels of the markers in a sample may be determined by comparison to a suitable "control" or "reference" sample. For example, the relative expression level of markers in a particular tumor may be determined with reference to the expression level of the same markers in a number of tumors of the same general class. Alternatively, the expression level of the markers may be determined with reference to the expression level of the same markers in the same tumor prior to treatment. If the expression level of markers is greater or less than that of the reference, e.g. the average expression level of tumors of a particular type or the pre-treatment sample, markers expression may be said to be "increased" or "decreased", respectively. Additionally, it is possible that the expression levels may remain constant between the control or reference and the sample.

Samples for analysis in such methods can be any organ, tissue, cell, or cell extract isolated from a subject, such as a sample isolated from a mammal having cancer. For example, a sample can include, without limitation, cells or tissue (e.g., from a biopsy), blood, serum, tissue or fine needle biopsy samples, or any other specimen, or any extract thereof, obtained from a patient (human or animal), test subject, healthy volunteer, or experimental animal. A subject can be a human, rat, mouse, non-human primate, etc. A sample may also include sections of tissues such as frozen sections taken for histological purposes. A "sample" may also be a cell or cell line created under experimental conditions, that is not directly isolated from a subject.

In one aspect of this method, the RNA is isolated from a fixed, wax-embedded cancer tissue specimen of the patient. In another embodiment, the RNA is isolated from core biopsy tissue or fine needle aspirate cells. In yet another embodiment, the cancer is breast cancer, small-cell lung cancer or colorectal cancer.

As described in detail below, expression of the biomarkers within a cancer cell may be evaluated by any suitable means. For example, expression may be evaluated using DNA microarrays. Alternatively, RNA transcripts may be measured using real time PCR, or, when RNA corresponds to a coding gene, protein products (total or post-translationally modified forms) may be detected using suitable antibodies. Methods of determining expression levels of genes by these and other methods are known in the art.

In the interest of brevity, Applicants are not expressly listing every possible combination of gene products suitable for use in the methods of the invention. Nevertheless, it should be understood that every such combination is contemplated and is within the scope of the invention. It is specifically envisioned that any combination of gene products listed in Tables 3-5 that were found to be differentially expressed between a control or reference, for example the untreated tumors, and the post-treatment tumors, may be particularly useful for analysis.

In one aspect of the invention, the markers may be evaluated in tumor tissue obtained from a patient treated with chemotherapy preferably between 24h and 21 days following the start of treatment. Increased expression of the markers is predictive of sensitivity and response to treatment, whereas lack of increase in marker expression is predictive of resistance or lack of response to treatment.

Biomarkers expression may be evaluated on the residual tumor tissue present in the surgical specimen obtained from a patient that received neoadjuvant chemotherapy Differential expression of the biomarkers may help to predict tumor relapse and to identify specific adjuvant therapy.

To determine the (increased, decreased) expression levels of the above described biomarkers in the practice of the present invention, any method known in the art may be utilized. In one preferred embodiment of the invention, expression based on detection of RNA which hybridizes to a "probe" or "primer" specific for the biomarkers described herein is used. A "probe" or "primer" is a single-stranded DNA or RNA molecule of defined sequence that can base pair to a second DNA or RNA molecule that contains a complementary sequence (the target). The stability of the resulting hybrid molecule depends upon the extent of the base pairing that occurs, and is affected by parameters such as the degree of complementarity between the probe and target molecule, and the degree of stringency of the hybridization conditions. The degree of hybridization stringency is affected by parameters such as the temperature, salt concentration, and concentration of organic molecules, such as formamide, and is determined by methods that are known to those skilled in the art.

Probes or primers specific for the nucleic acid biomarkers described herein, or portions thereof, may vary in length by any integer from at least 8 nucleotides to over 500 nucleotides depending on the purpose for which, and conditions under which, the probe or primer is used. Probes or primers specific for the nucleic acid biomarkers described herein may have greater than 20-30% sequence identity, or at least 55-75% sequence identity, or at least 75-85% sequence identity, or at least 85-99% sequence identity, or 100% sequence identity to the nucleic acid biomarkers described herein. Probes or primers may be derived from genomic DNA or cDNA, for example, by amplification, or from cloned DNA segments, and may contain either genomic DNA or cDNA sequences representing all or a portion of a single gene from a single individual. Probes or primers may be designed to bind selectively to transcript isoforms reflecting alternative splicing events such as those set forth in Table 4. Probes or primers may be chemically synthesized.

A probe or primer may hybridize to a nucleic acid biomarker under high stringency conditions as described herein. "Stringent hybridization conditions" as used herein mean conditions under which a first nucleic acid sequence (e.g., probe) will hybridize to a second nucleic acid sequence (e.g., target), such as in a complex mixture of nucleic acids. Stringent conditions are sequence-dependent and will be different in different circumstances. Stringent conditions may be selected to be about 5-10°C lower than the thermal melting point (Tm) for the specific sequence at a defined ionic strength and pH. The Tm may be the temperature (under defined ionic strength, pH, and nucleic concentration) at which 50% of the probes complementary to the target hybridize to the target sequence at equilibrium (as the target sequences are present in excess, at Tm, 50% of the probes are occupied at equilibrium). Stringent conditions may be those in which the salt concentration is less than about 1.0 M sodium ion, such as about 0.01-1.0 M sodium ion concentration (or other salts) at pH 7.0 to 8.3 and the temperature is at least about 30°C for short probes (e.g., about 10-50 nucleotides) and at least about 60° C for long probes (e.g., greater than about 50 nucleotides). Stringent conditions may also be achieved with the addition of destabilizing agents such as formamide. For selective or specific hybridization, a positive signal may be at least 2 to 10 times background hybridization. Exemplary stringent hybridization conditions include the following: 50% formamide, 5XSSC, and 1% SDS, incubating at 42°C, or, 5XSSC, 1% SDS, incubating at 65°C, with wash in 0.2XSSC, and 0.1% SDS at 65°C.

Probes or primers can be detectably-labeled, either radioactively or non-radioactively, by methods that are known to those skilled in the art. By "detectably labeled" is meant any means for marking and identifying the presence of a molecule, e.g., an oligonucleotide probe or primer, a gene or fragment thereof, or a cDNA molecule. Methods for detectably-labeling a molecule are well known in the art and include, without limitation, radioactive labeling (e.g., with an isotope such as 32P or 35S) and nonradioactive labeling such as, enzymatic labeling (for example, using horseradish peroxidase or alkaline phosphatase), chemiluminescent labeling, fluorescent labeling (for example, using fluorescein), bioluminescent labeling, or antibody detection of a ligand attached to the probe. Also included in this definition is a molecule that is detectably labeled by an indirect means, for example, a molecule that is bound with a first moiety (such as biotin) that is, in turn, bound to a second moiety that may be observed or assayed (such as fluorescein-labeled streptavidin). Labels also include digoxigenin, luciferases, and aequorin.

Probes or primers can be used in biomarker detection methods involving nucleic acid hybridization, such as nucleic acid sequencing, nucleic acid amplification by the polymerase chain reaction (e.g., RT-PCR), single stranded conformational polymorphism (SSCP) analysis, restriction fragment polymorphism (RFLP) analysis, Southern hybridization, northern hybridization, in situ hybridization, electrophoretic mobility shift assay (EMSA), fluorescent in situ hybridization (FISH), and other methods that are known to those skilled in the art.

A preferred embodiment using a nucleic acid based assay to determine biomarker expression is by immobilization of one or more biomarker sequences identified herein on a solid support, including, but not limited to, a solid substrate as an array or to beads or bead based technology as known in the art. Alternatively, solution based expression assays known in the art may also be used. The immobilized sequence(s) may be in the form of polynucleotides as described herein such that the polynucleotide would be capable of hybridizing to a DNA or RNA corresponding to the biomarker sequence(s).

The immobilized polynucleotide(s) may be used to determine the biomarker expression signature in a sample isolated from a subject having cancer. The immobilized polynucleotide(s) need only be sufficient to specifically hybridize to the corresponding nucleic acid molecules derived from the sample (and to the exclusion of detectable or significant hybridization to other nucleic acid molecules).

In embodiments where only one or a few biomarkers are to be analyzed, the nucleic acid derived from a sample isolated from a subject having cancer may be preferentially amplified by use of appropriate primers such that only the genes to be analyzed are amplified to reduce contaminating background signals from other genes expressed in the cancer cells. Alternatively, and where multiple genes are to be analyzed or where very few cells (or one cell) is used, the nucleic acid from the sample may be globally amplified before hybridization to the immobilized polynucleotides. Of course RNA, or the cDNA counterpart thereof may be directly labeled and used, without amplification, by methods known in the art.

A biochip may be used in the practice of the invention. The biochip may comprise a solid substrate comprising an attached probe or plurality of probes described herein. The probes may be capable of hybridizing to a target sequence under stringent hybridization conditions. The probes may be attached at spatially defined sites on the substrate. More than one probe per target sequence may be used, with either overlapping probes or probes to different sections of a particular target sequence. The probes may be capable of hybridizing to target sequences associated with a single disorder appreciated by those in the art. The probes may either be synthesized first, with subsequent attachment to the biochip, or may be directly synthesized on the biochip.

The solid substrate may be a material that may be modified to contain discrete individual sites appropriate for the attachment or association of the probes and is amenable to at least one detection method. Representative examples of substrates include glass and modified or functionalized glass, plastics (including acrylics, polystyrene and copolymers of styrene and other materials, polypropylene, polyethylene, polybutylene, polyurethanes, TeflonJ, etc.), polysaccharides, nylon or nitrocellulose, resins, silica or silica-based materials including silicon and modified silicon, carbon, metals, inorganic glasses and plastics. The substrates may allow optical detection without appreciably fluorescing.

Biomarker expression may also be measured based on detection of a presence, increase, or decrease in protein levels or activity may also be used. Antibody based detection methods are well known in the art and include sandwich and ELISA assays as well as Western blot and flow cytometry based assays as non-limiting examples. Antibodies for use in such methods of detection include polyclonal antibodies and monoclonal antibodies that specifically bind to the biomarkers of Tables 3 and/or 4. Such antibodies, as well as fragments thereof (including but not limited to Fab fragments) function to detect such biomarkers in cancer cells by virtue of their ability to specifically bind to such polypeptides to the exclusion of other polypeptides to produce a detectable signal. Recombinant, synthetic, and hybrid antibodies with the same ability may also be used in the practice of the invention.

The present invention provides a more objective set of criteria, in the form of gene expression profiles of a discrete set of genes, to discriminate (or delineate) between cancer outcomes. In particularly preferred embodiments of the invention, the assays are used to discriminate between responders and non-responders to chemotherapy.

### 5.3. PATIENT TREATMENT

The present invention provides methods for the selection of an appropriate cancer "treatment regimen" and predicting the outcome of the same. As used herein the phrase "treatment regimen" refers to a treatment plan that specifies the type of treatment, dosage, schedule and/or duration of a treatment provided to a subject in need thereof (e.g., a subject diagnosed with cancer). The selected treatment regimen can be an aggressive one which is expected to result in the best clinical outcome (e.g., complete cure of the disease) or a more moderate one which may relieve symptoms of the disease yet results in incomplete cure of the disease. The type of treatment can include a surgical intervention, administration of a therapeutic drug, an exposure to radiation therapy and/or any combination thereof. The dosage, schedule and duration of treatment can vary, depending on the severity of disease and the selected type of treatment, and those of skill in the art are capable of adjusting the type of treatment with the dosage, schedule and duration of treatment.

The identified drug-induced biomarker expression patterns in early post-treatment (24h to 21 days) and residual tumor cells from tumors of patients treated with chemotherapy may form the basis for a decision to apply a specific regimen for treatment of the subject. Thus, in this aspect, the present invention provides a method for treatment of a cancer in a subject in need thereof, comprising the steps of: a) measuring the amount and intensity of biomarker expression present in a tumor sample derived from a subject, and determining a sample value corresponding to said measurements; b) comparing the sample value obtained in step a) with a reference value, and depending on the sample/reference ratio obtained (greater than, equal to, or less than 1), c) treating said subject with the specific treatment regimen identified for each of the three classes, i.e., greater than, equal to, or less than 1.

Marker-negative tumors during the post-treatment phase (24h to 21 days) are predicted to be resistant to chemotherapy, and patients with marker-negative tumors can be spared the adverse side effects of a treatment that is unlikely to be beneficial. When available, alternative treatment can be administered accordingly. Conversely, marker-positive tumors are likely to be responsive to chemotherapy. Patients with marker-positive tumors can benefit from the addition of a treatment that targets the associated oncogenic/protective mechanisms activated in the marker-positive tumors as detailed further below. Tailoring treatment to the patient based on marker status will likely result in both cost savings and toxicity sparing by eliminating administration of ineffective treatments, and in improved clinical outcome by implementing specific adjuvant treatment based on marker expression. In a specific embodiment of the invention, the biomarkers to be assayed for expression levels in a cancer cell include those genes regulated in the IFN/Stat signaling pathway. Still further, said cancer cells may be derived from breast tumors, lung tumors, or colorectal tumors, for example.

In one embodiment, if no activation of the IFN/Stat markers is detected in a breast tumor of a patient receiving adriamycin/cyclophosphamide (A/C)-based chemotherapy, then the A/C-based chemotherapy can be substituted by potentially more efficient chemotherapeutic regimens, such as but not limited to those containing capecitabine, 5-fluorouracile, taxanes, and/or methotrexate. In addition or alternatively, treatment aimed at activating the IFN/Stat signaling pathway as a whole or at activating specifically tumor suppressor components of the IFN/Stat signaling pathway in the tumor can be administered with an expected clinical benefit.

In another embodiment, if activation of the IFN/Stat markers is detected in a breast tumor of a patient receiving A/C-based chemotherapy, then specific adjuvant therapy targeting specifically oncogenic/protective components of the IFN/Stat signaling pathway can be administered concomitantly or sequentially to ongoing A/C-based chemotherapy, such as Parp inhibitors, resulting in improved therapeutic efficacy.

In another embodiment, if activation of the IFN/Stat markers is detected in a small-cell lung cancer tumor of a patient receiving chemotherapy based on etoposide, ifosfamide and/or cisplatin, then specific adjuvant therapy targeting specifically oncogenic/protective components of the IFN/Stat signaling pathway can be administered concomitantly or sequentially to ongoing chemotherapy, such as Parp inhibitors, resulting in improved therapeutic efficacy

In another embodiment, if activation of the IFN/Stat markers is detected in a colorectal tumor of a patient receiving chemotherapy based on irinotecan or platinum salts, then specific adjuvant therapy targeting specifically oncogenic/protective components of the IFN/Stat signaling pathway can be administered concomitantly or sequentially to ongoing chemotherapy, such as Parp inhibitors, resulting in improved therapeutic efficacy

In yet another embodiment, if activation of the IFN/STAT markers is present in residual tumor tissue obtained from a patient treated with chemotherapy whose tumor has regressed following treatment, then specific adjuvant therapy targeting specifically oncogenic/protective components of the IFN/Stat signaling pathway can be administered, such as PARP inhibitors, resulting in a lower risk of tumor relapse.

It is envisioned that, in addition to Parp inhibitors, other adjuvant therapeutic approaches may be developed that target other genes whose increased or decreased expression may have a protective role in tumors exposed to chemotherapeutic drugs. It is well within the ability of one skilled in the art, using the teachings provided herein, to identify additional genes and genes products having such properties within the list provided in Tables 3-5. Some examples of genes having such already documented functions are given in the examples below and include: CCL5, PARP9, IFI6, PARP14, PARP12, DTX3L, LCN2, IFITM1, IFIT2, LAMP3, BST3, IFI44, DDX58, SAMHD1, SAMD9, IFI27, MUC15.

Since our data establish a link between treatment-induced activation of the IFN/Stat pathway in several tumor types (eg. breast, lung, colon) and tumor response to a variety of chemotherapeutic agents with different mechanisms of action, used alone or in combination, it is further envisioned that these markers could be useful to predict response and adapt the treatment of other malignant conditions treated with other chemotherapeutic agents.

### 5.4. XENOGRAFT MODEL

The present invention provides a xenograft model system for identifying a biomarker expression signature that is correlated with drug response and clinical outcome. The system comprises (i) developing a xenograft model showing response to therapy followed by tumor relapse, (ii) identifying genes differentially expressed between the residual and pre-treatment tumor wherein the differentially expressed genes, i.e., biomarkers, form a drug response expression signature, (iii) determining the drug response expression signature status of tumors from a population of humans, and (iv) correlating the resistance expression signature status with drug response and clinical outcome. The xenograft model system of the invention may also be used to identify novel chemotherapeutic compounds that may be used to treat cancer.

The invention relates to xenograft model system of human cancer, in particular, in mammals which carry transplanted human tumor cells. The invention also relates to the use of such xenograft model system in the study of cancer, particularly for evaluating candidates for chemotherapy. Thus, in one aspect, the invention provides an animal model of cancer, comprising a mammal which is immunodeficient and which carries a tumor xenograft. Preferably, the mammal is a mouse or rat. Tumor cell lines which may be used in this model include but are not limited to cells from solid tumors, such as those present in cancer of the colon, breast or lung. The tumor is preferably of human origin. It is particularly preferred that the tumors are introduced into the model of the invention by direct subcutaneous grafting of surgical specimen.

In a particular aspect of this embodiment, the chemotherapeutic agent is administered to the animal model of the present invention by any means known in the art including topical, oral or systemic.

The efficacy of the chemotherapeutic agent is determined by determining the drug response expression signature status of biomarkers of tumors before and after drug treatment. In an embodiment of the invention said biomarkers comprise those biomarkers regulated by the IFN/Stat1 signaling pathway. Biomarkers whose expression is predictive of cell-resistance to chemotherapy include the following: DTX3L, CCL5, IFIT1, IFITM1, IRF9, IFI6, IFI44, IFI44L, OAS1, OAS2, LAMP3, MX1, PARP9, PARP12, PARP14, SAMD9, SAMD9L, BST2, DDX60, CLDN1,STAT1, STAT2, UBE2L6, ZNFX1. Still another useful set of biomarkers is composed of STAT1, OAS1, LAMP3, IFI44 and CCL5.

### 5.5. KITS

A kit is also provided and may comprise a reagent for detection of a differentially expressed biomarker described herein together with any or all of the following: assay reagents, buffers, probes and/or primers, and sterile saline or another pharmaceutically acceptable emulsion and suspension base. In addition, the kits may include instructional materials containing directions (e.g., protocols) for the practice of the methods described herein. The kit may further comprise a software package for data analysis of expression profiles.

In a specific embodiment of the invention, the kits may include one or more reagents corresponding to the biomarkers described herein, e.g., antibodies that specifically bind the biomarkers or nucleic acid probes or primers that hybridize to the biomarkers, etc. In some embodiments, the kits may include a plurality of reagents, e.g., on an array, corresponding to the biomarkers described herein. The kits may include detection reagents, e.g., reagents that are detectably labeled. The kits may include written instructions for use of the kit, and may include other reagents and information such as control or reference standards, wash solutions, analysis software, etc.

The kits and arrays can be used to measure biomarkers according to the invention, to determine the likelihood that a cancer patient will respond to chemotherapy. The kits can also be used to monitor a subject's response to cancer therapy, enabling the medical practitioner to modify the treatment based upon the results of the test. The kits can also be used to identify and cancer therapeutics, such as small molecules, peptides, etc.

This invention will be better understood by reference to the Experimental Details that follow, but those skilled in the art will readily appreciate that these are only illustrative of the invention and should not be construed as limiting the scope of the invention. Additionally, throughout this application, various publications are cited. The disclosure of these publications is hereby incorporated by reference into this application to describe more fully the state of the art to which this invention pertains.

### 6. EXAMPLES

### 6.1 MATERIAL AND METHODS

**6.1.1 Animals and establishment of tumor xenografts.** Tumor xenografts were generated by direct subcutaneous grafting into immunodeficient mice of human tumor surgical samples with informed written consent of the patients and maintained by serial transplantation. The tumor xenografts have been studied for histology, cytogenetics, genetic and other biological markers, and for their response to a number of anticancer agents, alone and in combination. These studies have shown that tumor xenografts are biologically similar to the patient's tumors from which they derive, in term of both molecular characteristics and response to therapy. They are thus clinically relevant tumor models, as opposed to xenografts produced from tumor cell lines that have been obtained by in vitro culture and often passaged for many years (Fiebig, H.H. et al. Eur J Cancer 40, 802-820, 2004; Marangoni E., Vincent-Salomon A., Auger N. et al. Clin Cancer Res. 2007 Jul 1;13:3989-98).

**6.1.2 *In vivo* studies.** Chemotherapeutic drugs were administered by intraperitoneal route according to the following doses and schedules: adriamycin, 2 mg/kg, q3wk (Doxorubicin®, Teva Pharmaceuticals, France) and Cyclophosphamide, 100 mg/kg, q3wk (Endoxan®, Baxter, France). Irinotecan (Campto®, Pfizer Holding France), 50 mg/kg, q4dx4. Etoposide 12 mg/kg, qdx3. Ifosfamide 90 mg/kg, qdx3. bPARP inhibitor (AZD2281, Sequoia Research Products, UK) at 50 mg/kg (ip; qdx14). A second treatment cycle was performed with PARP inhibitor at 50 mg/kg (ip qdx5) during the nodule phase. Other pharmacological inhibitors are tested and administrated at the nodule phase: JAK1/2 Inhibitor (CP-690550) at 15 mg/kg (po qdx14), Epigallocatechin gallate (ECGC) (Sequoia Research Products, UK) at 30 mg/kg (ip qdx28), HDAC inhibitor (Trichostatine A), Retinoic acid at 50 mg/kg (ip qdx28) (Sigma-Aldrich, France). Breast tumor xenograft models were transplanted into 5-week old female Swiss nude mice, as described above, one tumor being transplanted into 5-10 recipients. When tumors reached a volume of 60-250 mm3, mice were individually identified and randomly assigned to the control or treated groups (12 to 20 mice per group). Tumor volume was evaluated by measuring two perpendicular diameters of the tumor, with a calliper, biweekly during the treatment period and once a week during the follow-up period. The formula TV (mm3) = [length (mm) x width (mm)2]/2 was used, where the length and the width were the longest and the shortest diameters of the tumor, respectively. All animals were weighted biweekly during the treatment period and the follow-up period. Mice were ethically sacrificed when the tumor volume reached 2000 mm3. Individual tumor growth delays (TGD) is calculated as the time in days required for individual tumors to reach 3- to 5-fold the initial median tumor volume. Median growth delay /group was calculated and reported in the tables. The tumor growth delay index (TGDI) is calculated as the median growth delay in the treated group divided by the median growth delay in the control group. The percentage ratio between the mean tumor volume of a treated group (T) and the mean tumor volume of the control group (C) is calculated.

**6.1.3. Tumor sampling.** Tumors were collected from 5 independent mice at different time points: prior to treatment (= controls with volume < 200 mm3); post-treatment residual tumors (= nodules with volume < 32 mm3 until minimal palpation); recurrent tumors (= regrowth with volume > 62.5 mm3). Tumors or nodules were processed for (1) FFPE (formalin-fixed paraffin-embedded) or for (2) snap frozen samples: samples were cut into 3-4 mm pieces (at least 3 fragments) and snap-frozen in liquid nitrogen, then transferred to -80C° for storage. Samples were allocated for histology, microdissection and RNA analyses.

**6.1.4. Cryosectioning.** Blocks were taken out of the deep-freezer (-80°C) individually when needed and transferred into the cryotome (-30°C) without thawing. Each withdrawal was recorded. Multiple 10 µm thick sections were placed onto PALM FrameSlides PET: 2 slides for Laser Capture Microdissection (LCM); 1 slide as backup after Cresyl Violet staining (if necessary) and 2 sections transferred directly into a microfuge tube as internal control (if necessary). Slides were stained with Cresyl Violet according to the PALM protocol. Controls were frozen back at - 80°C, slides for LCM were stored at -20°C until usage or used directly after drying.

**6.1.5. Laser Capture Microdissection (LCM).** The sample FrameSlide was put on a thin glass slide (0,17mm) as support and placed on the microscope stage of a PALM MicroBeam IV system. Appropriate areas (= elements) for LCM were selected in the 5x- and 10x- objective until an area of 2x 4 Mio µm² was reached. In each AdhesiveCap (opaque 500µl) an area of 4 Mio µm² was transferred by the RoboLPC function of the MicroBeam. Images were taken automatically before and after the transfer process. Energy and focus were adjusted to the needs of the sample whenever necessary and were recorded within the individual image headers automatically by the system. AdhesiveCaps with captured samples were assembled with according tubes, provided with 350µL Qiazol solution and incubated for 30min. Tubes were rocked over-head at room temperature for lysis and subsequently frozen at -20°C until RNA extraction. Microdissected areas of 4 µm2 were transferred for RNA extraction using the Qiagen miRNeasy kit according to the manufacturer's instructions (Qiagen). Total RNA extracted was quantitatively and qualitatively assessed with the Bioanalyzer. From each eluate 1 µL was taken for quality analysis. Prior application onto the BioAnalyzer RNA 6000 PicoChip this aliquot was diluted 1:10 to ensure optimal electrophoresis (avoid overloading).

**6.1.6. Histology analysis.** The morphology of xenografts' tumor tissues was studied using paraffin-embedded sections and standard protocols. Tumor samples were fixed in 10% formalin for 2 to 5 days, placed in ethanol 70% and paraffin embedded (FFPE blocks). Paraffin-embedded sections, 4-µm thick, were used for light microscopy examination after haematoxylin-eosin-safran (HES) staining to differentiate the tumoral components and for immunohistochemical studies.

**6.1.7. Microarray analyses of gene expression and exon splicing.** Gene expression analysis at gene and exon level from tumor tissue was assessed by using the Affymetrix GeneChip® Human Exon 1.0 ST Array platform. The starting amount of total tumor RNA used for each reaction was 100 ng. Microarray hybridization and data normalization were outsourced to GenoSplice Technology, a biotech with expertise in transcriptional profiling. Genosplice Technology provided statistical analysis of the data to detect gene lists and functional gene classes associated with sample comparison. These analyses were repeated and implemented at XenTech by using BRB array tools, a bioinformatic package for microarray analysis.

**6.1.8. miRNA qPCR profiling.** miRNA biomarker analyses were performed on the miRCURY LNA™ Universal RT microRNA PCR platform, outsourced to Exiqon service (Exiqon, Denmark). This system is a microRNA-specific, LNA™-based PCR-based system designed for sensitive and accurate detection of microRNA by qPCR using SYBR® Green. For each RT reaction 40 ng total RNA was used. Three RT replicates per sample were used for real-time amplification on ready-to-use microRNA PCR Human Panel I and II run on a LightCycler-480 real-time PCR system. Analyses were performed for a set of 742 human microRNAs. Average Cq values were normalized to three stably expressed reference genes using the Exiqon GenEx software.

**6.1.9. Real-time quantitative RT-PCR.** Total RNA amount was measured using the Nanodrop (ND1000, ThermoFisher) and RNA quality was assessed with the Agilent 2100 Bioanalyzer onto RNA NanoChip (Agilent Technology, Massy, France).Three samples from each condition were pooled. Two µg of total RNA were reverse-transcribed with the Transcriptor First strand cDNA synthesis kit (Roche Applied Science, Switzerland) according to manufacturer's instructions. For the priming method, a combination of ¾ random hexamer primers and ¼ anchored-oligo(dT)18 primers was used to avoid 3'-prime bias in cDNAs. qPCR analyses of gene transcripts was done with 5 ng of RNA for each reaction. Three housekeeping genes were used as references for relative quantification of target genes expression. Oligonucleotides sequences used for PCR amplifications are listed in Table1 (HPRT1, GAPDH, RPL13, BST2, CLDN1, DDX60, IFI6, IFI44, IFI44L, IFIT1, IFITM1, IRF9, LAMP3, MX1, OAS1, OAS2, PARP9, PARP12, SAMD9, SAMD9L, STAT1, STAT2, ZNFX1).

**6.1.10. IFN/Stat protein expression evaluation by western blotting.** Protein expression was measured from tumor samples lysates extracted in non-denaturing lysis buffer (Tris-HCL 50 mM, pH7.5, Triton X-100 0.1%, NaCL 150 mM, EDTA 1 mM, Hepes 50 mM, NaF 1 mM, Na3VO4 2mM, protease inhibitor cocktail (Roche Diagnostics, Mannheim, Germany) . Quantification of total protein was done in order to load equivalent protein quantity (30 □g). Samples were boiled in NuPAGE LDS sample loading buffer (Invitrogen, Carlsbad, CA) containing 2-mercaptoethanol 5% (Sigma Chemical Co). Lysates were separated by 4 - 12% NuPAGE Novex Bis-Tris Mini Gels (Invitrogen, Carlsbad, CA) and transferred to nitrocellulose membranes (Whatman Inc, Sanford, US). Membranes were blocked for 1 h with 3% bovine serum albumine (BSA, Sigma-Aldrich, St. Louis, MO) in PBS 1X/0.1% Tween20. Then, membranes were probed with primary antibodies against phospho-Stat1 Tyr701, Total Stat1 (1/750-1000; Santa Cruz antibodies, Santa Cruz, CA), phospho-Stat1 Ser727 (1/1000; Merck Millipore, Billerica, MA), Oas1, Ifi44, Ifi27 (1/1000; Sigma-Aldrich, St. Louis, MO) or actin (1/1000; Sigma-Aldrich, St. Louis, MO) as a reference, in the same working solution, overnight at 4°C. After washing, membranes were incubated with horseradish-peroxidase-conjugated secondary antibody, directed against the species of primary antibody (Pierce), during 1h at room temperature in 2% nonfat dry milk/PBS 1X/0.1% Tween20. Immunoreactive bands were visualized using supersignal Femto chemiluminescence reagent (Pierce, Rockford, IL). Membranes were scanned using Fx7 camera system (Fusion Molecular Imaging Fx7, Viller Lourmat, France).

### 6.2 RESULTS

**6.2.1 Identification of tumor xenograft models showing tumor relapse following chemotherapy-induced tumor regression - (Drug response profile).** To identify tumor xenograft models that showed incomplete tumor eradication, characterized by residual tumor cell foci after chemotherapy treatment, antitumor activity of conventional cytotoxic drugs was tested in a panel of human cancer xenograft models (Table 2). Three categories of responders were defined based on the TGDI and T/C values. 1) High responders (HR) in which the treatment induced complete regressions (with minimal tumor palpation after treatment); 2) responders (R) in which T/C was inferior to 42% and TGDI superior to 2 fold, respectively; and 3) non-responders in which the growth parameters were not significantly altered by the treatment (T/C > 42% and TGDI < 2). All breast tumor models had been characterized for their response to chemotherapy. On a total of 17 tumors, eight were highly responders to A/C combination treatment with complete regression, eight responders and nine no responders. These models were used based on their documented high frequency of complete tumor regressions after chemotherapy, followed by tumor recurrence. Only the eight highly responder models and few responders shared these criteria defined as follows: induction of complete tumor regressions (tumor size < to 32 mm3) in the majority of treated animals; regrowth for 80% of tumors; presence of residual tumor cells after regression validated by histology. Our study focused on HBCx-6 and -10, highly responders to A/C with T/C = 1.31 and 0.55% at D21 and two responders, HBCx-8 and -17 with T/C = 33.86 and 16.42%, respectively (Table 2). An example of in vivo tumor regression-regrowth assay is shown in Figure 1. A human breast cancer xenograft, HBCx-6, treated with the combination A/C therapy, showed complete tumor regressions in 96% of treated mice after 19 days of treatment, followed by a tumor recurrence (Table 2). Similar profiles of "complete" tumor response, followed by late recurrence were obtained with other breast tumor xenografts treated with A/C combination. Of note, similar tumor regressions were obtained when cyclophosphamide was used as single agent instead of combined with Adriamycin (Table 2). In the same way, other types of tumor xenografts (brain, lung, colon) using a variety of conventional therapeutic agents showed this profile of response. Results based on the assessment of nodules criteria are shown in Table 2. The delay of nodule appearance was 14 days (HBCx-6), 18 days (HBCx-8), 28 days (HBCx-10) and 30 days (HBCx-17) after A/C treatment. More than 90% of nodules were obtained in HBCx-6 and HBCx-10 and 30% in HBCx-8 after one cycle of A/C treatment. The frequency of nodules increased after two cycles of treatment: 100% in HBCx-6 and HBCx-10; 57% in HBCx-8 and 30% in HBCx-17. Globally, the frequency of nodules and the relapse delay were increased in highly responder compared to responder tumors.

**6.2.2. Histological Characterization of the Residual Tumor Cells Responsible for Chemoresistance.** To characterize the residual tumor foci responsible for chemoresistance, histology analysis was performed and showed small foci of tumor cells within a stromal matrix and strong necrotic areas. In some areas, an abundant murine stroma presented fibroblasts with infiltrating inflammatory areas. Tumor cells presented a typical morphology (large cytoplasm with hyperchromatic nucleus). To isolate the human tumor foci from the murine stroma, laser-capture microdissection was performed. All samples (untreated, residual tumor foci and recurrent tumors) were treated in the same conditions. After cresyl violet staining of the sections to differentiate the human tumor components in twelve sets of samples, 3 breast cancer tumor models were micro-dissected for RNA extraction (HBCx-6, 8, 17). All these tumor models allowed transcriptome analysis, exon splicing and miRNA profiling.

**6.2.3 Investigation of new Diagnostic Markers and Therapeutic Targets linked to Chemoresistance by Genomic Approach.** To identify novel diagnostic markers and putative therapeutic targets linked to chemoresistance, parental tumors prior to treatment (A) were compared to residual tumor foci (B). The Exon array technology provided information not only on the overall expression level of every single annotated gene, but also on the transcriptional isoforms preferentially expressed for each gene due to alternative transcription start site or splicing events. The identification of genes differentially expressed between untreated growing tumor and residual tumors was performed in 3 models (Table 3). Residual tumor nodules of three analyzed breast cancer models, HBCx-6, 8 and 17 showed consistent overexpression of several genes belonging to the IFN/Stat pathway, a molecular sensor of intracellular stress inducible by various factors such as viral infection, X-ray irradiation, cytokines and toxic metabolites. In order to confer further robustness to the analysis, data from untreated control tumors were pooled and compared to data from their corresponding residual tumor cells in the 3 models. The gene list obtained was used to identify pathways significantly deregulated in the two conditions. This analysis revealed a consistent up-regulation of many genes involved in the IFN pathway, as well as a gene cluster related to the pathway, such as the Jak-Stat and Toll-like receptor pathways. In addition, the lists of biomarkers contain many RNA transcripts and micro-RNA with unknown function or previously undocumented functional relationship with tumor response to chemotherapy.

The identification of genes associated with cellular response to stress, survival and senescence in residual tumor cells that survived chemotherapy suggests a role for these pathways in cell resistance to drug-induced cell death. Additional evidence of IFN/Stat pathway activation in residual cells that resist treatment was provided by the observation that a significant fraction of genes overexpressed in the residual tumor foci bear DNA consensus motifs for binding to proteins of the Stat and Irf (interferon-responsive factor) family. It is known from the literature that these transcription factors act as transcriptional activators of target genes in response to activation of the IFN/Stat pathway. Analysis of available functional annotations for the top differentially (up)-regulated genes between the post-treatment residual tumors and the untreated tumors identified several interesting genes/pathways that could be involved in chemoresistance through their anti-apoptotic (survival and senescence mechanisms) or DNA repair function. A few interesting candidates identified in the gene list are listed below:

IFI6 (G1P3) is interferon-regulated and has been shown to play a role in protection from apoptosis. IFI6/G1P3 has been implicated in resistance to TRAIL-mediated apoptosis in myeloma cells (Cheriyath V et al. G1P3, an IFN-induced survival factor, antagonizes TRAIL-induced apoptosis in human myeloma cells. J Clin Invest 17: 3107-3117, 2007). It probably acts at the mitochondrial level on the release of cytochrome-c, but its mechanism of action is still unknown.

PARP9 (BAL1) and DTX3L (BBAP) genes are located in a head-to-head orientation and are co-regulated by the same γ-IFN-responsive bidirectional promoter. PARP9 belongs to the subfamily of macro-PARPs and is catalytically inactive, while DTX3L is an E3 ligase. BBAP and BAL1 are most abundant in a subtype of diffuse large B cell lymphoma (DLBCL) characterized by a prominent inflammatory infiltrate, increased γ-IFN production and an aggressive phenotype. Interestingly, it was recently discovered that a BAL1/BBAP protein complex localizes to the nucleus where it participates in the repair of doxorubicin-induced DNA damage (Yan Q et al. Mol Cell 36: 110-120, 2009). BBAP, together with its partner BAL1, was found to confer protection from doxorubicin-induced DNA damage in the HEK293 human transformed embryonic kidney cell line. DNA damage initiates a cascade of cellular signaling events that culminate in either the repair of DNA breaks or apoptosis. This study shows that BBAP/BAL1-induced histone H4 ubiquitylation, together with other histone modifications (methylation and acetylation) plays a key role in this process by regulating the binding of check point mediators such as 53BP1 and BRCA1, which allow DNA repair to take place. Our observation of up-regulation of PARP9 and DTXL3 in breast and lung tumor cells surviving chemotherapy provides an indication that these proteins participate in resistance to chemotherapy of cancer cells by increasing their DNA damage repair capability.

PARP14 (BAL2), like PARP9, also belongs to the IFN-regulated macro-PARPs family. While PARP9 is catalytically inactive, PARP14 possesses mono-(ADP-ribosyl)ation activity. In the mouse, PARP14 was found to play a major role in mediating protection against apoptosis in IL4-treated B-cells, including that after DNA damage (Cho SH et al. Blood 113: 2416-2425, 2010). Its mechanism of action is not completely understood, but it has been shown to regulate the expression of pro-survival factors at the transcriptional level.

CCL5 (RANTES) is an IFN-regulated chemokine whose expression is associated with cancer progression (Hembruff SL et al. Cancer Ther. 7:254-267, 2009). CCL5 mediates many types of tumor-promoting cross-talks between tumor cells and cells of the tumor microenvironment. Together, the overall current information indicates that CCL5 is an inflammatory mediator with pro-malignancy and pro-metastatic activities in breast and other cancers. However, its role in chemo-resistance had not yet been described.

**6.2.4 RNA splicing variants.** The transcriptional isoforms due to alternative splicing events (cassette exons, alternative promoters or splicing), were analyzed in the same experimental conditions. The purpose was to detect transcriptional isoforms differentially expressed in two different experimental conditions (untreated versus residual tumor) and consequently, provided additional set of potential diagnostic markers. The genes listed in Table 4 (including RPL32, UBXD7, IF6, MX1, TP53BP1) showed up-regulation of specific exons in residual tumor cells (Table 4).

**6.2.5. MicroRNA biomarkers.** By determining the expression of 738 miRNAs, patterns of differentially expressed miRNA could be discerned in untreated versus residual tumors after chemotherapy. Differentially expressed miRNAs provide additional diagnostic markers and potential therapeutic targets for chemoresistant residual disease (Table 5).

**6.2.6. Time-course qPCR validation of the gene/microRNA expression signature detected in residual tumor cells and assessment of the gene/microRNA signature in early post-treatment tumor samples.** Validation of the microarray gene and microRNA expression data was performed by qPCR using primers specific for human sequences (eg. non-cross-reactive with murine sequences). The effect of chemotherapy on the activation of 21 genes implicated in IFN/Stat pathway (IFIT1, IFITM1, IRF9, IFI6, IFI44, IFI44L, OAS1, OAS2, LAMP3, MX1, PARP9, PARP12, SAMD9, SAMD9L, BST2, DDX60, CLDN1, STAT1, STAT2, UBE2L6, ZNFX1) and two miRNA (miR-142-3p and miR150) was analyzed in responder versus non-responder tumor models. A time-course analysis was performed in human breast, colon or small-cell lung cancer xenografts following A/C, CPT-11 or VP16/ifosfamide treatment respectively (Fig. 3A-C). Tumors were collected at the following time points: D0 (pre-treatment = control), D1, D3, D7, at the nodule and regrowth phases. Increased gene expression was observed in responder (HBCx-6, HBCx-8, HBCx-10, HBCx-14, HBCx-15, HBCx-17, TC301 and SC61) but not in non-responder (HBCx-2, HBCx-12B, HBCx-16, HBCx-24, HBCx13A) tumors. The following genes: LAMP3, CLDN1, MX1, IFIT1, DDX60, OAS1, UBE2L6 and STAT1 were significantly overexpressed in responder tumors compared to non-responder tumors. Similarly, gene expression was globally increased in the residual nodules of a colorectal tumor treated with CPT-11 and in a small-cell lung cancer tumor treated with the VP16/ifosfamide combination. In the same way, miR-142-3p expression was increased at day-7 in responder (HBCx-6 and HBCx-17) compared to non-responder tumors (HBCx-2 and HBCx-12B) (Fig.3E). Globally, these results show that increased expression of IFN/Stat pathway target genes is an early process that occurs specifically in drug-responsive tumors following administration of chemotherapy and can still be detected at the residual nodule stage following drug-induced tumor regression. This observation implies that tumor suppressor components of the IFN/Stat signaling pathway contribute to the antitumor effect of chemotherapy in drug-responsive tumors.

**6.2.7. Time-course analysis of STAT1 expression in early post-treatment tumor cells.** Tumors were collected at the following time points: D0 (pre-treatment = control), D3, D7 and analyzed by western blotting for Stat1 expression (Fig. 3D and 4). Stat1 has two isoforms activated by IFNs. Stat1 was phosphorylated at both tyrosine 701 and serine 727 residues. The Tyr 701 site is preferentially phosphorylated by JAK1, which promotes the dimerization and the translocation of Stat1 in the nucleus. The Ser727 site is phosphorylated by MAPKp38 and detected in Statla; this site may be required for a maximal induction of Stat1-mediated gene activation. Globally, the amount of phosphorylated and total Stat1 protein was increased in responder (HBCx-6, HBCx-10, HBCx-14, and HBCx-17) but not in non-responder (HBCx-2, HBCx-12B, HBCx-16, HBCx-24) tumors. Expression was highly increased between 3 and/or 7 days after treatment in responder tumors. Expression of the Stat1 gene transcript was positively correlated with STAT1 protein expression and phosphorylation (Fig. 4A). Treatment with cyclophosphamide alone resulted in a similar increase in total and phosphorylated Statlprotein than treatment with the A/C combination, consistent with their similar antitumor efficacy (Table 2).

**6.2.8. Validation of gene/miRNA/protein expression signature in residual tumor cells.** To validate potential markers and putative therapeutic targets linked to tumor relapse, parental tumors prior to treatment (C) were compared to residual tumor foci (N) and regrowths (R) in different xenograft tumor models. Residual tumor foci showed an increase in expression of genes related to the IFN/Stat signaling pathway and in protein expression of at least Stat1, Ifi27, Ifi44, Lcn2 and Oas1 compared to untreated tumors (Fig.3D). Micro-RNAs were found differentially expressed between residual tumor cells and untreated tumors. Expression of two miR-142-3p and miR-150 was measured by qPCR and showed increased expression in residual tumor cells compared to untreated tumors (Fig. 3E and F). Analyses of tumors that regrew following chemotherapy-induced complete regression indicated a decrease in the expression of genes or proteins relative to the residual tumor cells, suggesting that these alterations were transient.

**6.2.9. Functional validation of molecular targets and identification of "druggable" pathways to achieve tumor eradication in xenograft models.** Functional studies were performed to examine whether these genes and their corresponding pathways could be used as therapeutic targets to overcome tumor chemo-resistance. An example is given with the HBCx-6 breast cancer xenograft model that combined conventional (A/C) chemotherapy and drugs to selectively inhibit one of the mechanisms presumably activated in A/C-treated tumor cells. In combination groups, A/C was administrated concomitantly with the PARP inhibitor AZD2281at D0 (Fig. 5). A second cycle of treatment was performed at the nodule phase (minimal tumor palpation) with AZD2281. A/C combination (at 2/100 mg/kg) induced strong anti-tumor activity resulting in complete tumor regression in 10 out of 12 tumors (T/C = 12.21% at D21), followed by tumor recurrence in all tumors. AZD2281 at 50 mg/kg used as single agent demonstrated significant anti-tumor activity in the HBCx-6 model, with only 2 out of 7 tumors showing complete tumor regression. However, the combination of A/C with AZD2281 had much improved anti-tumor activity compared to A/C treatment alone (T/C = 7.7% at D21). Moreover, the follow-up period revealed a significant difference in term of delay or frequency of tumor regrowth in the A/C-AZD2281 combination group compared to the A/C treatment group. In particular, tumor relapse was completely prevented in mice receiving a second treatment cycle of PARP inhibitor at the nodule stage.

**6.2.10. Immunohistochemistry and paraffin-Q-PCR.** Evaluation of IFN/STAT pathway activation in tumor cells within tumors exposed to chemotherapy is performed by immunohistochemistry. Several candidate biomarkers are tested using commercial antibodies (IFITM1, IFI44, IFI44L, IFI6, IFIT2, IFI27, MX1, OAS1, PARP9, PARP14, BST2, STAT1, phospho-STAT1, CCL5, DTX3L, MUC15, LCN2, SAMD9, SAMD9L). The objective is to establish a robust IHC scoring system in order to facilitate the validation of the xenograft observations in retrospective cohorts of cancer patients for whom FFPE samples are available. As another approach, a method of Q-RT-PCR scoring performed on RNA extracted from FFPE samples is developed.

**6.2.11. Validation of Tumor Biomarkers in human breast cancer samples.** Analyses of clinical tumor specimen collections from patients with documented clinical responses are performed in collaboration with clinical centers. Retrospective analysis of tumor samples from breast cancer patients explores the relationship between the presence of an activated IFN/STAT pathway, bad prognosis and response to chemotherapy. Paired clinical FFPE samples are tested at different steps (before chemotherapy and at the residual disease stage after neoadjuvant treatment) for the expression level of candidate biomarkers.

**6.2.12. Functional validation of therapeutic targets in tumor xenograft models.** This approach combines conventional therapy and selected drugs to inhibit the mechanisms that are activated in residual tumor cells and potentially involved in protection from DNA damage-induced cell death. The IFN/STAT pathway is directly or indirectly inhibited (suppressors of INF/STAT pathway or PARP, HDAC, RARα inhibitors) using pharmacological or genetic siRNA (directed against over-expressed genes of the IFN/STAT pathway, such as those described above) approaches. This results in developing therapies combining specific pharmacological or genetic inhibitors and chemotherapy able to kill tumor cells surviving chemotherapy and responsible for relapses in malignancies. Such pharmacological inhibitors include but are not limited to JAK1/2 inhibitors, Epigallocatechin gallate, histone deacetylases inhibitor (Trichostatine), which targets the pro-DNA repair activity of PARP9 and DTX3L, or retinoic acid (RARα). These drugs are administrated during the tumor regression or nodule phase after chemotherapy treatment in order to increase the antitumor efficacy of chemotherapy, and prevent or delay tumor recurrence in human tumor xenograft models. The role of specific gene products and pathways (e.g. apoptosis, DNA repair) in resistance to chemotherapy is evaluated in vitro in cell culture model systems. siRNA gene knockout experiments are performed in vitro in tumor-derived primary cell cultures and/or cell lines with an active or induced IFN/STAT pathway. Different models are used to test the role of specific gene implicated in chemoresistance in vitro then in vivo: IC20DAN (a primary NSCLC tumor xenograft) which shows high constitutive activation of STAT signaling and cell lines (Hela, MCF7 and MDA MB231) in which STAT1 can be activated by treatment with IFN-γ. Results show two classes of IFN-induced genes: STAT1-dependent (UBE2L6, IFI44L, OAS2, PARP12, IF44, STAT2, PARP9, BST2) and independent (IFIT1, IFIT3, OAS1, LAMP3, MX1, IFITM1). Once specific gene candidates are identified by siRNA gene knock-down experiments in vitro, lentiviral vector carrying short hairpin RNAs (shRNA) are used for target validation in vivo in tumors treated with chemotherapy with tumor regression and relapse as a read-out.

**Table 1. Primer sequences for qPCR**

| **Gene** | **F Primer (5'--> 3')** | **R Primer (5'-->3')** |
|---|---|---|
| Hs HPRT1 | GCTTTCCTTGGTCAGGCAGTATAAT | AAGGGCATATCCTACAACAAACTTG |
| | (SEQ ID No. 1) | (SEQ ID No. 2) |
| Hs GAPDH | CCACATCGCTCAGACACCAT | CCCAATACGACCAAATCCGT |
| | (SEQ ID No. 3) | (SEQ ID No. 4) |
| Hs RPL13 | CCCGTCCGGAACGTCTATAA | CTAGCGAAGGCTTTGAAATTCTTC |
| | (SEQ ID No. 5) | (SEQ ID No. 6) |
| Hs IFIT1 | AGGTTCTCCTTGCCCTGAA | AAAGCCCTATCTGGTGATGC |
| | (SEQ ID No. 7) | (SEQ ID No. 8) |
| Hs IFI44L | CATGATGAAACCCCATCTCC | CTGTAGCCTCCACCTCCAAG |
| | (SEQ ID No. 9) | (SEQ ID No. 10) |
| Hs OAS2 | ACAGCTGAAAGCCTTTTGGA | GCATTAAAGGCAGGAAGCAC |
| | (SEQ ID No. 11) | (SEQ ID No. 12) |
| Hs IFIT3 | ATCAGCGCTACTGCAACCTT | TGCAGCAGATCTCCATTCTG |
| | (SEQ ID No. 13) | (SEQ ID No. 14) |
| Hs OAS1 | GAGAAGGCAGCTCACGAAAC | AGGAGGTCTCACCAGCAGAA |
| | (SEQ ID No. 15) | (SEQ ID No. 16) |
| Hs IFI44 | AGCCTGTGAGGTCCAAGCTA | TTTGCTCAAAAGGCAAATCC |
| | (SEQ ID No. 17) | (SEQ ID No. 18) |
| Hs IFI6 | AGGATGAGGAGTAGCCAGCA | TTGGGAGGTTGAGACAGGAG |
| | (SEQ ID No. 19) | (SEQ ID No. 20) |
| Hs LAMP3 | CCCAACAACTCACACACAGC | CTGGAAGGGTGGTCTGGTTA |
| | (SEQ ID No. 21) | (SEQ ID No. 22) |
| Hs MX1 | ACCTACAGCTGGCTCCTGAA | CGGCTAACGGATAAGCAGAG |
| | (SEQ ID No. 23) | (SEQ ID No. 24) |
| Hs PARP9 | TCTCCAGAACCACCACATCA | CCTTGCCATTTCCTCCTGTA |
| | (SEQ ID No. 25) | (SEQ ID No. 26) |
| Hs SAMD9 | GTGCAAGGATCCCAGACAGT | AGCTTTGCTTCCTTGGTGAA |
| | (SEQ ID No. 27) | (SEQ ID No. 28) |
| Hs BST2 | AGGTGGAGCGACTGAGAAGA | GGAATGTTCAAGCGAAAAGC |
| | (SEQ ID No. 29) | (SEQ ID No. 30) |
| Hs DDX60 | CCCAGGGTCCAGGATTTTAT | GAACAGTTGCTGCCACTTGA |
| | (SEQ ID No. 31) | (SEQ ID No. 32) |
| Hs CLDN1 | CCGTTGGCATGAAGTGTATG | CCAGTGAAGAGAGCCTGACC |
| | (SEQ ID No. 33) | (SEQ ID No. 34) |
| Hs IFITM1 | CAACACTTCCTTCCCCAAAG | CCAGACAGCACCAGTTCAAG |
| | (SEQ ID No. 35) | (SEQ ID No. 36) |
| Hs STAT1 | GCTGCTCCTTTGGTTGAATC | TGCTCCCAGTCTTGCTTTTC |
| | (SEQ ID No. 37) | (SEQ ID No. 38) |
| Hs UBE2L6 | ACCCTTCCCACACCCTTTAC | CCATCTGTCTCCCACCCATA |
| | (SEQ ID No. 39) | (SEQ ID No. 40) |
| Hs IRF9 | GCCATTCTGTCCCTGGTGTA | CAGTGTGTGCGAGGATTTTC |
| | (SEQ ID No. 41) | (SEQ ID No. 42) |
| Hs PARP12 | CCTCCTCTTTTTGTCCCACA | CTCCCATTTGCCTCTATCCA |
| | (SEQ ID No. 43) | (SEQ ID No. 44) |
| Hs STAT2 | GAGCACCAGGATGATGACAA | GATTCGGGGATAGAGGAAGC |
| | (SEQ ID No. 45) | (5EQ ID No. 46) |
| Hs ZNFX1 | ATGCCCAGGTTGTAGGAATG | CCCAATCAAAATGAGGTGCT |
| | (SEQ ID No. 47) | (SEQ ID No. 48) |

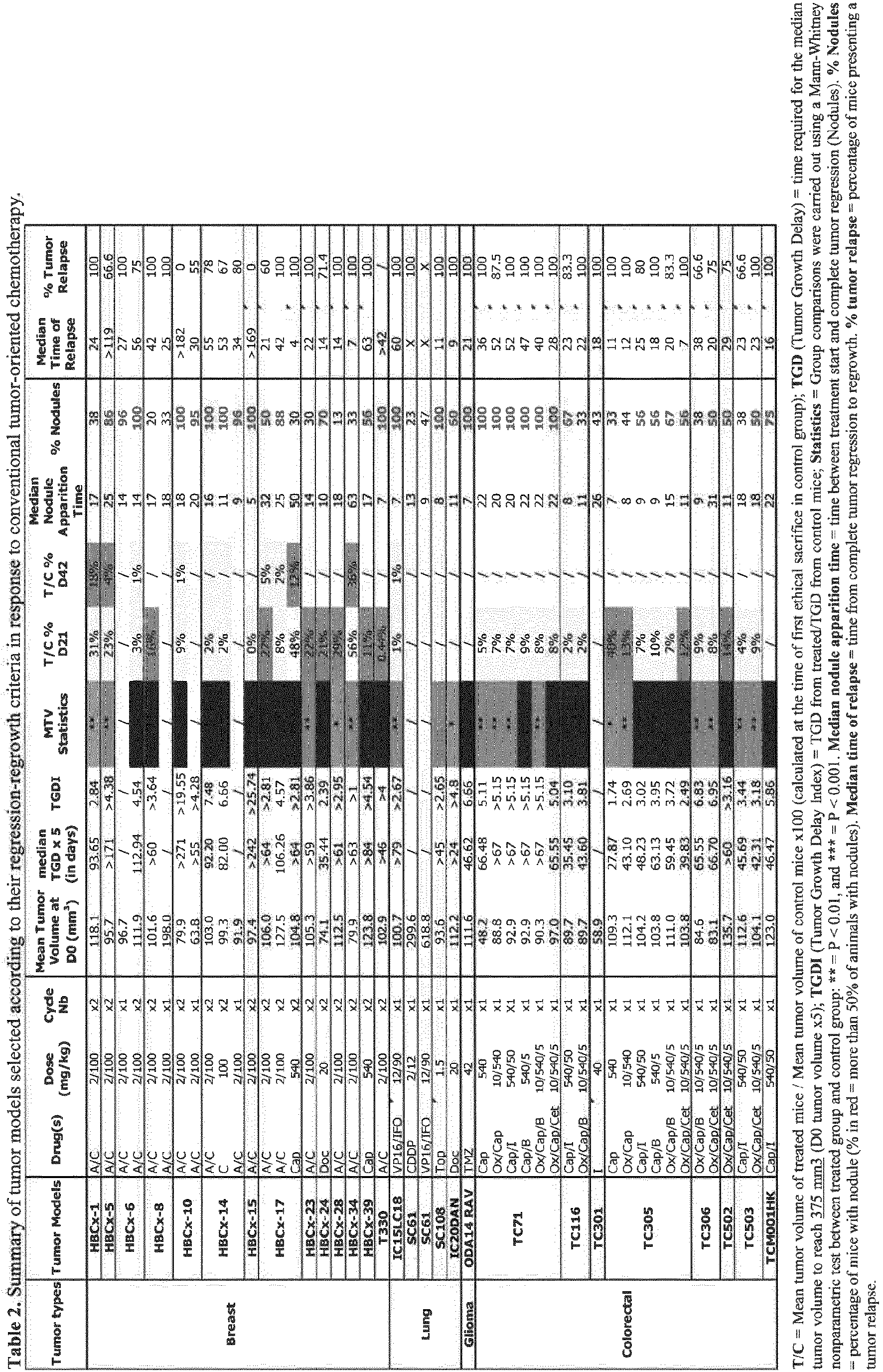

**Table 3. List of genes differentially expressed between residual tumor cells (nodules) and untreated tumors. Data were pooled from three experiments (HBCx-6, HBCx-8 and HBCx-17), N/T ratio > 1.5 or < 0.67 ; p < 5x10e-3. Genes listed in Table 1 are in bold. *IFN targets are defined according to the paper published by Khodarev et al, PNAS 101: 1714-1719, 2004.**

| **p value** | **FDR** | **Nodules (N)** | **Untreated Tumors (T)** | **N/T ratio** | **Unique id** | **Gene symbol** | **IFN targets*** |
|---|---|---|---|---|---|---|---|
| **< 1e-07** | **< 1e-07** | **178.85** | **27.18** | **6.580206** | **2343473** | **IFI44L** | |
| < **1e-07** | < **1e-07** | 80.05 | 16.03 | 4.9937617 | 3257192 | IFIT2 | |
| **1.00E-07** | **0.000734** | **403.76** | **100.68** | **4.0103298** | **2707876** | **LAMP3** | + |
| **2.00E-07** | **0.0011** | **212.51** | **33.6** | **6.3247024** | **3432514** | **OAS2** | |
| **3.00E-07** | **0.0011** | **277.61** | **63.18** | **4.3939538** | **2692060** | **PARP9** | |
| 3.00E-07 | 0.0011 | 240.23 | 108.53 | 2.2134894 | 3753860 | CCL5 | |
| **9.00E-07** | **0.00283** | **211.69** | **29.08** | **7.2795736** | **3257246** | **IFIT1** | + |
| 1.30E-06 | 0.00358 | 97.22 | 51.14 | 1.9010559 | 3474831 | OASL | |
| **2.30E-06** | **0.00563** | **235.59** | **142.23** | **1.6564016** | **3457752** | **STAT2** | |
| 4.80E-06 | 0.0096 | 601 | 1039.61 | 0.5781014 | 3114832 | SQLE | |
| **5.70E-06** | **0.0105** | **263.69** | **54.71** | **4.819777** | **3432438** | **OAS1** | + |
| **7.30E-06** | **0.0124** | **196.79** | **110.7** | **1.7776874** | **3529701** | **IRF9** | |
| 1.10E-05 | 0.0161 | 135.9 | 43.55 | 3.1205511 | 2735362 | HERC6 | |
| **1.22E-05** | **0.0164** | **422.79** | **224.49** | **1.8833356** | **3373962** | **UBE2L6** | |
| **1**.**42E**-**05** | **0.0164** | **79.96** | **21.06** | **3.7967711** | **3854454** | **BST2** | + |
| **1.52E-05** | **0.0164** | **442.99** | **101.22** | **4.3765066** | **3922100** | **MX1** | + |
| **1.53E-05** | **0.0164** | **71.53** | **15.46** | **4.6267788** | **3257204** | **IFIT3** | |
| **1.56E-05** | **0.0164** | **68.48** | **17.69** | **3**.**8711136** | **2343511** | **IFI44** | + |
| **1**.**77E**-**05** | **0.0172** | **29.97** | **11.54** | **2.5970537** | **2792800** | **DDX60** | |
| 1.82E-05 | 0.0172 | **78.21** | **37.78** | **2.0701429** | **2531377** | SP100 | |
| **1.88E-05** | **0.0172** | **3473.08** | **987.69** | **3.5163665** | **2403261** | **IFI6** | |
| 2.27E-05 | 0.0192 | 148.26 | 93.58 | 1.5843129 | 2982319 | SOD2 | |
| **2.36E-05** | **0.0192** | **545.25** | **235.96** | **2.310773** | **2592268** | **STAT1** | + |
| 2.44E-05 | 0.0192 | 69.7 | 27.57 | 2.5281103 | 2468351 | RSAD2 | |
| **2.68E-05** | **0.0203** | **42.01** | **22.21** | **1.8914903** | **3257268** | **IFIT5** | |
| 3.10E-05 | 0.0221 | 117.25 | 69.15 | 1.6955893 | 3442475 | C1R | |
| 3.11E-05 | 0.0221 | 218.31 | 85.96 | 2.5396696 | 3432467 | OAS3 | + |
| 3.34E-05 | 0.023 | 93.39 | 168.26 | 0.5550339 | 3470272 | | |
| 3.77E-05 | 0.0251 | 137.72 | 39.21 | 3.5123693 | 2639054 | PARP14 | |
| 5.80E-05 | 0.0365 | 188.48 | 100.69 | 1.871884 | 2699726 | PLSCR1 | + |
| 6.30E-05 | 0.0385 | 17.37 | 10.59 | 1.6402266 | 2754937 | TLR3 | |
| 6.51E-05 | 0.0387 | 200.5 | 71.73 | 2.7952042 | 3203086 | DDX58 | |
| 6.77E-05 | 0.0392 | 109.76 | 168.25 | 0.6523626 | 3994710 | MAMLD1 | |
| 0.0001084 | 0.0508 | 38.02 | 22.83 | 1.6653526 | 2334602 | TSPAN1 | |
| 0.0001248 | 0.0561 | 62.68 | 23.75 | 2.6391579 | 3904691 | SAMHD1 | |
| 0.000151 | 0.0639 | 394.28 | 237.34 | 1.6612455 | 2437118 | MUC1 | |
| 0.0001587 | 0.0647 | 38.12 | 20.44 | 1.8649706 | 3360142 | TRIM21 | |
| 0.0001979 | 0.0739 | 185.15 | 120.14 | 1.5411187 | 3820310 | C19orf66 | |
| 0.0002016 | 0.0739 | 10.33 | 15.86 | 0.6513241 | 3551774 | | |
| 0.0002115 | 0.0739 | 139.45 | 51.89 | 2.6874157 | 2638962 | DTX3L | |
| **0.0003197** | **0.101** | **120.06** | **28.39** | **4.2289539** | **3061438** | **SAMD9** | |
| 0.0003941 | 0.114 | 45.62 | 26.09 | 1.7485627 | 3218528 | ABCA1 | |
| 0.0004045 | 0.114 | 74.64 | 48.34 | 1.5440629 | 2844313 | | |
| **0.0004272** | **0.118** | **156.29** | **89.89** | **1.7386806** | **3908831** | **ZNFX1** | |
| 0.0004412 | 0.118 | 40.47 | 25.81 | 1.5679969 | 3722338 | IFI35 | + |
| 0.0005033 | 0.126 | 1027 | 680.33 | 1.5095615 | 3957679 | SELM | |
| 0.000573 | 0.14 | 165.83 | 259.71 | 0.6385199 | 3676684 | | |
| 0.0006154 | 0.145 | 217.85 | 75.96 | 2.8679568 | 3549575 | IFI27 | + |
| **0.0006971** | **0.157** | **2731.27** | **598.59** | **4.5628393** | **3315675** | **IFITM1** | |
| 0.0007578 | 0.162 | 178.8 | 60.54 | 2.9534192 | 3366903 | MUC15 | |
| 0.0007658 | 0.162 | 280.29 | 155.71 | 1.8000771 | 3936550 | USP18 | |
| 0.0007726 | 0.162 | 100.08 | 25.97 | 3.8536773 | 3061456 | SAMD9L | |
| 0.0010201 | 0.191 | 37.09 | 20.55 | 1.8048662 | 3725950 | | |
| 0.0011521 | 0.199 | 18.08 | 36.12 | 0.5005537 | 3435879 | | |
| 0.0012513 | 0.203 | 99.84 | 57.8 | 1.7273356 | 3181193 | TDRD7 | |
| 0.0012551 | 0.203 | 324.66 | 101.85 | 3.1876289 | 3848039 | C3 | |
| **0.0012646** | **0.203** | **285.67** | **139.47** | **2.0482541** | **3075932** | **PARP12** | |
| 0.0012813 | 0.204 | 58.13 | 98.91 | 0.587706 | 3740594 | | |
| 0.0014747 | 0.215 | 68.5 | 38.15 | 1.7955439 | 2603051 | SP110 | |
| 0.0016479 | 0.225 | 18.21 | 32.96 | 0.5524879 | 3470324 | | |
| 0.0016593 | 0.225 | 71.48 | 46.42 | 1.5398535 | 2748346 | TLR2 | |
| 0.0016758 | 0.226 | 15.61 | 34.92 | 0.4470218 | 3096512 | | |
| 0.0017661 | 0.231 | 113.96 | 53.75 | 2.120186 | 2364231 | DDR2 | |
| 0.001827 | 0.237 | 179.65 | 273.68 | 0.6564236 | 3831227 | | |
| 0.0019932 | 0.246 | 107.16 | 71.1 | 1.507173 | 3458587 | DDIT3 | |
| 0.0024306 | 0.258 | 33.36 | 21.21 | 1.572843 | 2735409 | HERC5 | |
| 0.0025868 | 0.265 | 431.5 | 651.41 | 0.6624092 | 2318823 | | |
| 0.0026877 | 0.268 | 1649.31 | 2625.12 | 0.6282799 | 2947081 | HIST1H4L | |
| 0.0027085 | 0.269 | 26.81 | 16.58 | 1.6170084 | 3403168 | C1S | |
| 0.0028562 | 0.278 | 166.03 | 258.8 | 0.6415379 | 2968317 | | |
| 0.0029163 | 0.283 | 35.91 | 15.65 | 2.2945687 | 3318443 | TRIM22 | |
| 0.0030205 | 0.288 | 69.23 | 105.68 | 0.6550908 | 2968331 | | |
| 0.0030881 | 0.291 | 77.02 | 38.12 | 2.0204617 | 2584207 | IFIH1 | |
| **0.0033792** | **0.304** | **680.79** | **244.76** | **2.7814594** | **2710599** | **CLDN1** | |
| 0.0037342 | 0.316 | 6.44 | 12.71 | 0.5066876 | 3570109 | | |
| 0.0040953 | 0.333 | 7.45 | 11.34 | 0.6569665 | 2775508 | | |
| 0.0042056 | 0.339 | 424.59 | 676.96 | 0.627201 | 2946268 | HIST1H2BC | |
| 0.0043472 | 0.344 | 877.3 | 273.99 | 3.2019417 | 3592023 | B2M | |
| 0.0044266 | 0.344 | 287.86 | 160.23 | 1.7965425 | 3708858 | CD68 | |
| 0.0045448 | 0.345 | 28.25 | 17.97 | 1.5720646 | 3944243 | APOL6 | |
| 0.004861 | 0.355 | 382.3 | 250.73 | 1.5247477 | 2901620 | HLA-E | |
| 0.0049388 | 0.356 | 21.6 | 35.09 | 0.61556 | 2841446 | | |

**Table 4. List of exon splicing and genes differentially expressed between residual nodule cells (N) and untreated tumors (T). Data were pooled from three experiments (HBCx-6, HBCx-8 and HBCx-17). Exon over-expression cut-off is > 1.5; under-expression cut-off is < 0.67, p value cut-off is <5x10e-4.**

| **exon** | **Symbol** | **NvsT ratio** | **p value ex** |
|---|---|---|---|
| e3 | RPL32 | 0.54 | 4.30E-08 |
| e18 | RAB6IP1 | 0.65 | 5.51E-08 |
| e21 | ARHGAP29 | 1.36 | 6.23E-07 |
| e12 | RAPGEF6 | 1.66 | 7.00E-07 |
| e4 | UBXD7 | 1.72 | 7.46E-07 |
| e4 | MFN2 | 1.36 | 9.66E-07 |
| e19 | TP53BP1 | 1.87 | 1.30E-06 |
| e2 | RPL32 | 0.54 | 2.26E-06 |
| e4 | TAOK2 | 1.58 | 4.08E-06 |
| e6 | SMURF1 | 0.63 | 4.56E-06 |
| e13 | KCTD3 | 0.81 | 1.56E-05 |
| e2 | FAM175B | 1.92 | 1.62E-05 |
| e1 | GMPPA | 0.48 | 1.66E-05 |
| e4 | RPL32 | 0.55 | 2.12E-05 |
| e5 | OS9 | 1.69 | 2.22E-05 |
| e3 | FURIN | 0.67 | 2.29E-05 |
| e13 | PARP14 | 1.50 | 2.33E-05 |
| e24 | RNF31 | 1.80 | 2.36E-05 |
| e9 | HOOK3 | 1.66 | 2.45E-05 |
| e14 | UBA1 | 0.66 | 3.52E-05 |
| e8 | RBCK1 | 0.83 | 3.74E-05 |
| e3 | SGK1 | 1.20 | 3.89E-05 |
| e20 | NAV2 | 1.34 | 3.92E-05 |
| e11 | ANKRD13A | 1.51 | 3.98E-05 |
| e18 | MYCBP2 | 1.30 | 4.07E-05 |
| e2 | MARK2 | 1.75 | 4.28E-05 |
| e20 | FER1L3 | 1.25 | 4.35E-05 |
| e9 | CLPTM1 | 0.66 | 4.41E-05 |
| e14 | REV3L | 0.64 | 5.58E-05 |
| e13 | SMC3 | 1.77 | 5.74E-05 |
| e7 | RBBP8 | 0.67 | 5.94E-05 |
| e9 | RANGAP1 | 0.79 | 5.97E-05 |
| e51 | DYNC1H1 | 0.82 | 6.47E-05 |
| e17 | USP7 | 0.67 | 6.49E-05 |
| e36 | CHD3 | 0.67 | 7.81E-05 |
| e2 | COQ9 | 1.53 | 7.90E-05 |
| e4 | IFI6 | 0.52 | 8.03E-05 |
| e2 | IQGAP1 | 1.85 | 8.11E-05 |
| e4 | TRPM7 | 1.22 | 8.12E-05 |
| e18 | SF3B3 | 1.52 | 8.12E-05 |
| e25 | SP100 | 0.69 | 8.35E-05 |
| e27 | PDCD11 | 1.22 | 8.45E-05 |
| e8 | AAMP | 0.57 | 9.52E-05 |
| e16 | MX1 | 1.43 | 9.52E-05 |
| e9 | IK | 0.64 | 9.56E-05 |
| e13 | LMNA | 1.53 | 9.58E-05 |
| e2 | RPS21 | 1.79 | 9.59E-05 |
| e2 | CENPA | 1.63 | 9.73E-05 |
| e3 | DKFZp547E087 | 1.59 | 9.79E-05 |
| e7 | PSMC6 | 1.22 | 9.88E-05 |
| e4 | EEF2 | 0.79 | 1.01E-04 |
| e7 | GPX4 | 1.50 | 1.01E-04 |
| e7 | ZNFX1 | 0.76 | 1.01E-04 |
| e9 | VARS | 0.79 | 1.05E-04 |
| e10 | SFRS11 | 1.57 | 1.15E-04 |
| e2 | BTBD14B | 0.83 | 1.16E-04 |
| e6 | GATC | 1.52 | 1.17E-04 |
| e12 | GTF2I | 1.76 | 1.18E-04 |
| e13 | MYST2 | 0.75 | 1.23E-04 |
| e53 | PRKDC | 0.72 | 1.39E-04 |
| e4 | MLL5 | 1.68 | 1.40E-04 |
| e2 | C20orf24 | 0.79 | 1.55E-04 |
| e27 | SPAG9 | 1.27 | 1.56E-04 |
| e14 | NCOA3 | 0.66 | 1.59E-04 |
| e21 | KIDINS220 | 0.78 | 1.60E-04 |
| e28 | STAG1 | 0.70 | 1.60E-04 |
| e13 | CMIP | 1.52 | 1.62E-04 |
| e49 | TPR | 1.64 | 1.78E-04 |
| e4 | C1orf172 | 1.49 | 1.84E-04 |
| e6 | UCP2 | 1.75 | 1.85E-04 |
| e12 | PARP14 | 1.35 | 1.96E-04 |
| e23 | PARP1 | 1.49 | 1.96E-04 |
| e15 | GOLGB1 | 0.81 | 1.98E-04 |
| e29 | PLXNB1 | 1.69 | 2.15E-04 |
| e1 | B2M | 0.55 | 2.19E-04 |
| e6 | ITFG1 | 0.67 | 2.20E-04 |
| e13 | UTP15 | 0.67 | 2.22E-04 |
| e6 | ILDR1 | 0.63 | 2.22E-04 |
| e9 | PRKCD | 1.32 | 2.36E-04 |
| e3 | KIAA1128 | 0.65 | 2.38E-04 |
| e3 | FIP1L1 | 0.64 | 2.42E-04 |
| e24 | ITGA3 | 0.83 | 2.42E-04 |
| e7 | FAM62A | 1.23 | 2.45E-04 |
| e20 | ACTN1 | 0.81 | 2.56E-04 |
| e10 | MINA | 0.68 | 2.59E-04 |
| e31 | PDS5B | 0.81 | 2.64E-04 |
| e5 | sept-07 | 1.50 | 2.80E-04 |
| e27 | BRWD3 | 1.58 | 2.80E-04 |
| e35 | ZFYVE26 | 1.29 | 2.96E-04 |
| e21 | SPAG9 | 0.65 | 3.03E-04 |
| e4 | PGM3 | 1.33 | 3.15E-04 |
| e6 | GPHN | 1.55 | 3.20E-04 |
| e5 | YWHAE | 1.55 | 3.21E-04 |
| e22 | ITGA2 | 0.62 | 3.21E-04 |
| e3 | UBXD7 | 1.78 | 3.21E-04 |
| e27 | PLCB4 | 1.69 | 3.36E-04 |
| e29 | MON2 | 1.57 | 3.41E-04 |
| e1 | DYNLRB1 | 1.31 | 3.44E-04 |
| e8 | HSPA9 | 1.24 | 3.44E-04 |
| e11 | REV3L | 0.61 | 3.58E-04 |
| e7 | ERMP1 | 0.61 | 3.59E-04 |
| e8 | MELK | 0.79 | 3.60E-04 |
| e3 | STAT1 | 0.64 | 3.77E-04 |
| e3 | MRPL47 | 0.64 | 3.78E-04 |
| e4 | GALNT11 | 1.55 | 3.79E-04 |
| e19 | SEC31A | 0.80 | 3.82E-04 |
| e19 | MX1 | 1.22 | 3.83E-04 |
| e13 | ZDHHC20 | 1.76 | 3.85E-04 |
| e2 | VAC14 | 1.61 | 3.85E-04 |
| e10 | GTF2I | 1.63 | 3.98E-04 |
| e55 | MYCBP2 | 1.29 | 3.98E-04 |
| e6 | DHX40 | 0.65 | 3.99E-04 |
| e5 | STAT2 | 1.29 | 4.15E-04 |
| e15 | HGS | 0.65 | 4.16E-04 |
| e2 | CASC4 | 0.65 | 4.17E-04 |
| e2 | CCNT2 | 1.48 | 4.18E-04 |
| e22 | KIAA1033 | 1.21 | 4.20E-04 |
| e6 | KRT16 | 0.54 | 4.20E-04 |
| e3 | C5orf32 | 0.83 | 4.22E-04 |
| e5 | TBCE | 0.76 | 4.40E-04 |
| e15 | NBPF11 | 1.44 | 4.57E-04 |
| e10 | ANKRD13A | 1.72 | 4.58E-04 |
| e20 | LRPPRC | 1.54 | 4.62E-04 |
| e44 | CENPE | 0.74 | 4.63E-04 |
| e7 | RAB6IP1 | 0.62 | 4.81E-04 |
| e7 | RABGAP1L | 0.68 | 4.99E-04 |
| e5 | ABBA-1 | 1.57 | 5.00E-04 |

**Table 5. List of microRNAs differentially expressed between residual tumor cells (nodules) and untreated tumors (controls). Data were pooled from four experiments (HBCx-6, HBCx-8, HBCx-10 and HBCx-17). MiRNA intensity values correspond to relative quantification obtained by normalization of miRNA Cp with the average Cp of the assay.**

| a) Supervised analysis of Nodules vs Control samples, data filtered according to negative control threshold. This expression matrix contains raw data found to be at least 5 cross over points (Cps) below the negative controls. | | | | | | |
|---|---|---|---|---|---|---|
| **Order** | **p value** | **FDR** | **Nodules** | **Controls** | **Fold-change** | **Unique id** |
| 1 | 1,58E-05 | 0,00929 | 0,23 | 0,039 | 5,79 | hsa-miR-150 |
| 2 | 0,0002941 | 0,0865 | 0,69 | 0,19 | 3,68 | hsa-miR-223 |
| 3 | 0,0011967 | 0,235 | 0,29 | 0,14 | 2,13 | hsa-miR-29c |
| 4 | 0,0026712 | 0,393 | 1,06 | 0,66 | 1,59 | hsa-miR-30b |
| 5 | 0,0037716 | 0,444 | 6,06 | 2,76 | 2,2 | hsa-miR-21 |
| 6 | 0,0059632 | 0,479 | 0,11 | 0,042 | 2,62 | hsa-miR-140-5p |
| 7 | 0,0060151 | 0,479 | 0,57 | 0,23 | 2,46 | hsa-miR-140-3p |
| 8 | 0,006908 | 0,479 | 0,064 | 0,1 | 0,62 | hsa-miR-339-3p |
| 9 | 0,0088813 | 0,479 | 3,08 | 4,81 | 0,64 | hsa-miR-106a |
| 10 | 0,00992 | 0,479 | 0,08 | 0,14 | 0,57 | hsa-miR-18a |
| 11 | 0,0099216 | 0,479 | 0,032 | 0,057 | 0,55 | hsa-miR-505 |
| 12 | 0,0103676 | 0,479 | 3,74 | 5,49 | 0,68 | hsa-miR-20a |
| 13 | 0,0105936 | 0,479 | 0,91 | 0,48 | 1,92 | hsa-miR-29a |
| 14 | 0,0127222 | 0,508 | 0,037 | 0,022 | 1,67 | hsa-miR-199b-5p |
| 15 | 0,0145247 | 0,508 | 0,02 | 0,039 | 0,51 | hsa-miR-299-5p |
| 16 | 0,0147249 | 0,508 | 0,22 | 0,13 | 1,67 | hsa-miR-101 |
| 17 | 0,0154614 | 0,508 | 0,067 | 0,18 | 0,37 | hsa-miR-138 |
| 18 | 0,0163541 | 0,508 | 0,025 | 0,0094 | 2,62 | hsa-miR-125b-1* |
| 19 | 0,016422 | 0,508 | 0,0066 | 0,033 | 0,2 | hsa-miR-1179 |
| 20 | 0,0176391 | 0,519 | 0,11 | 0,063 | 1,8 | hsa-miR-29b |
| 21 | 0,0191238 | 0,535 | 0,42 | 0,65 | 0,65 | hsa-miR-324-5p |
| 22 | 0,0248398 | 0,664 | 0,011 | 0,035 | 0,31 | hsa-miR-135b* |
| 23 | 0,0267914 | 0,685 | 0,015 | 0,026 | 0,58 | hsa-miR-29b-2* |
| 24 | 0,0280763 | 0,688 | 0,13 | 0,21 | 0,62 | hsa-miR-17* |
| 25 | 0,0309963 | 0,7 | 0,016 | 0,039 | 0,4 | hsa-miR-760 |
| 26 | 0,031452 | 0,7 | 0,015 | 0,023 | 0,67 | hsa-miR-130b |
| 27 | 0,0321882 | 0,7 | 0,18 | 0,077 | 2,39 | hsa-miR-146a |
| 28 | 0,0336366 | 0,7 | 0,12 | 0,088 | 1,39 | hsa-miR-328 |
| 29 | 0,0349538 | 0,7 | 0,25 | 0,38 | 0,66 | hsa-miR-18b |
| 30 | 0,0356964 | 0,7 | 0,51 | 0,32 | 1,63 | hsa-miR-26a |
| 31 | 0,0395527 | 0,75 | 0,11 | 0,071 | 1,54 | hsa-miR-30a* |
| 32 | 0,0427107 | 0,777 | 0,11 | 0,068 | 1,65 | hsa-miR-148a |
| 33 | 0,0473337 | 0,777 | 1,7 | 1.11 | 1,54 | hsa-miR-34a |
| 34 | 0,0493533 | 0,777 | 0,88 | 1.29 | 0,69 | hsa-miR-196a |
| 35 | 0,0499126 | 0,777 | 0,029 | 0,064 | 0,45 | hsa-miR-23b* |

| b) Supervised analysis of Nodules vs Control samples, unfiltered data | | | | | | |
|---|---|---|---|---|---|---|
| **Order** | **p value** | **FDR** | **Nodules** | **Controls** | **Fold-change** | **Unique id** |
| 1 | 2,00E-07 | 0,00012 | 7,45 | 0,8 | 9,27 | hsa-miR-142-3p |
| 2 | 5,00E-06 | 0,00151 | 2,51 | 0,3 | 8,51 | hsa-miR-150 |
| 3 | 0,0002947 | 0,0591 | 7,41 | 1,9 | 3,91 | hsa-miR-223 |
| 4 | 0,0006501 | 0,0978 | 0,045 | 0,16 | 0,28 | hsa-miR-942 |
| 5 | 0.0009498 | 0,103 | 0,12 | 0,031 | 3,8 | hsa-miR-142-5p |
| 6 | 0,0010259 | 0,103 | 65,31 | 27,99 | 2,33 | hsa-miR-21 |
| 7 | 0,0023282 | 0,176 | 0,22 | 0,087 | 2,47 | hsa-miR-338-3p |
| 8 | 0,0025184 | 0,176 | 1,18 | 0,41 | 2,88 | hsa-miR-140-5p |
| 9 | 0,0026253 | 0,176 | 3,13 | 1,39 | 2,26 | hsa-miR-29c |
| 10 | 0.0031864 | 0,192 | 1,23 | 0,45 | 2,72 | hsa-miR-29b |
| 11 | 0,0076857 | 0,368 | 6,09 | 2,34 | 2,61 | hsa-miR-140-3p |
| 12 | 0,0084924 | 0,368 | 0,059 | 0,14 | 0,42 | hsa-miR-103-as |
| 13 | 0,0085029 | 0,368 | 11,42 | 6,75 | 1,69 | hsa-miR-30b |
| 14 | 0,0087703 | 0,368 | 2,38 | 1,35 | 1,77 | hsa-miR-101 |
| 15 | 0,009458 | 0,368 | 1,22 | 0,67 | 1,83 | hsa-miR-148a |
| 16 | 0,0098907 | 0,368 | 0,26 | 0,08 | 3,3 | hsa-miRPlus-A1031 |
| 17 | 0,0103892 | 0,368 | 0,063 | 0,38 | 0,17 | hsa-miR-217 |
| 18 | 0,0132068 | 0,442 | 0,033 | 0,15 | 0,22 | hsa-miR-885-3p |
| 19 | 0,0139357 | 0,442 | 1,98 | 0,65 | 3,06 | hsa-miR-146a |
| 20 | 0,0150017 | 0,452 | 0,02 | 0,12 | 0,17 | hsa-miR-518e |
| 21 | 0,0183777 | 0,525 | 0,2 | 0,42 | 0,48 | hsa-miR-299-5p |
| 22 | 0,0197314 | 0.525 | 9,85 | 4,83 | 2,04 | hsa-miR-29a |
| 23 | 0,0201052 | 0,525 | 0,17 | 0,37 | 0,47 | hsa-let-7f-1* |
| 24 | 0,0209158 | 0,525 | 0,021 | 0,088 | 0,24 | hsa-miR-548o |
| 25 | 0,0234088 | 0,564 | 0,11 | 0,047 | 2,29 | hsa-miR-145* |
| 26 | 0,0259775 | 0,592 | 0,092 | 0,25 | 0,37 | hsa-miR-196b |
| 27 | 0,0265738 | 0,592 | 18,38 | 11,27 | 1,63 | hsa-miR-34a |
| 28 | 0,0338924 | 0,684 | 5,38 | 1,9 | 2,83 | hsa-miR-424 |
| 29 | 0,0339199 | 0,684 | 0,14 | 0,25 | 0,55 | hsa-miR-29b-2* |
| 30 | 0,0341074 | 0,684 | 0,027 | 0,079 | 0,34 | hsa-miRPlus-C1089 |
| 31 | 0,0389448 | 0,756 | 0,69 | 1,11 | 0,63 | hsa-miR-339-3p |
| 32 | 0,0408184 | 0,768 | 0,38 | 0,22 | 1,7 | hsa-miR-199b-5p |

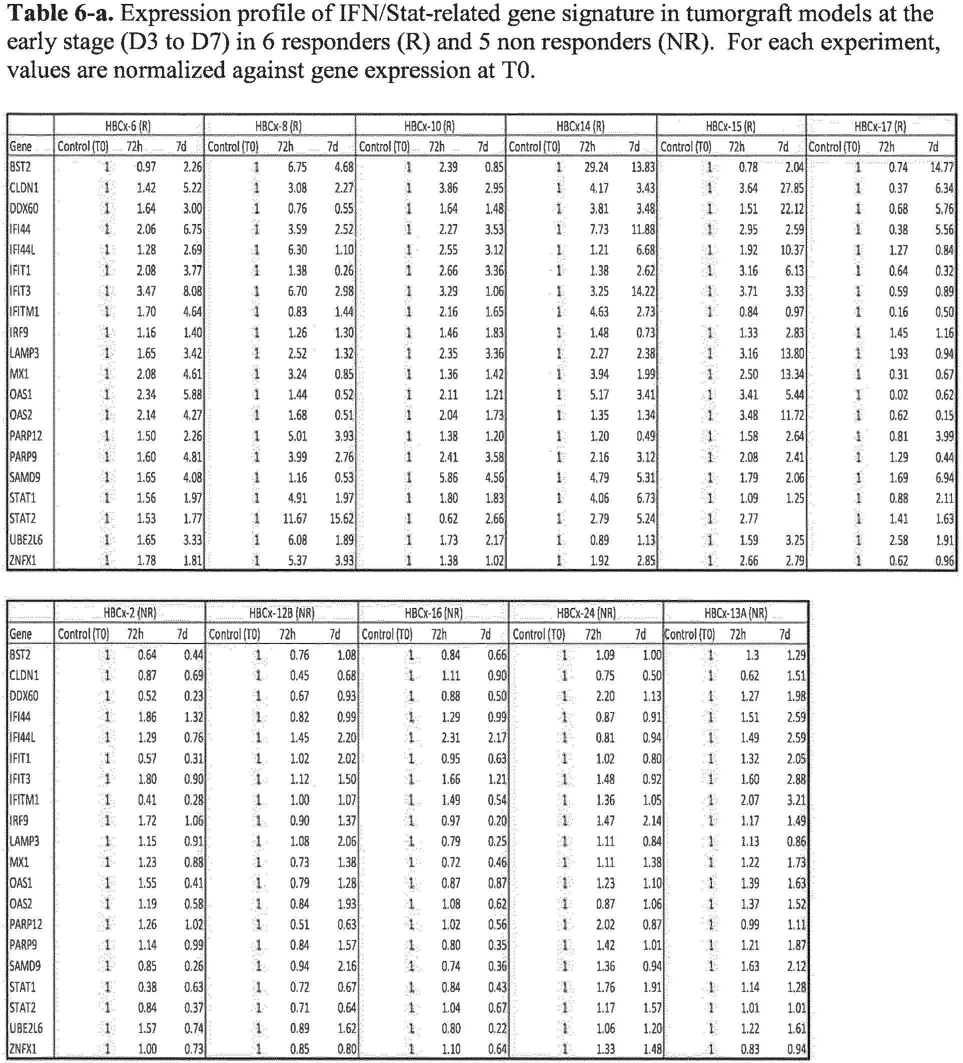

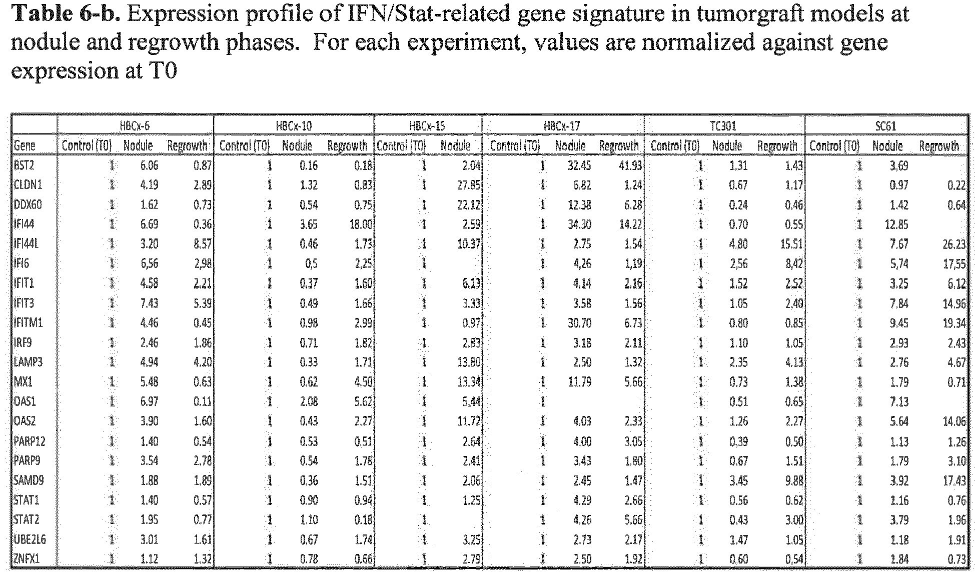

## Claims

1. A method of predicting tumor response in a patient subjected to chemotherapy comprising:
(i) measuring the amount of expression in a sample of cancer cells from a subject of a differentially expressed biomarker wherein said biomarker is selected from those differentially expressed biomarkers which are regulated by the IFN/STAT signaling pathway,
(ii) comparing the amount of expression with those of the same biomarker in the same tumor prior to chemotherapy, and
(iii) predicting the likelihood of a response to chemotherapy based on the expression of the biomarker.

2. The method of claim 1, wherein the biomarker is evaluated in sample of cancer cells obtained from a patient treated with chemotherapy between 24h and 21 days following the start of the chemotherapy.

3. The method of claim 2, wherein increased expression being predictive of sensitivity and response to chemotherapy, whereas lack of increase in expression being predictive of resistance or lack of response to chemotherapy.

4. The method of claim 1, wherein the biomarker is evaluated in sample of cancer cells obtained from residual tumor tissue present in the surgical specimen obtained from a patient that received neoadjuvant chemotherapy.

5. The method of claim 4, wherein differential expression of the biomarker is associated with cancer relapse following chemotherapy.

6. A method for treatment of a cancer in a subject treated with chemotherapy, comprising the steps of:
a) measuring the amount and intensity of biomarker expression present in a tumor sample derived from a subject, said biomarker being regulated by the IFN/STAT signaling pathway and determining a sample value corresponding to said measurements;
b) comparing the sample value obtained in step a) with a reference value, said reference value being the one of the same biomarker in the same tumor prior to chemotherapy, and depending on the sample/reference ratio obtained,
c) treating said subject with the specific treatment regimen.

7. The method of claim 6, wherein the specific treatment regimen is as follows:
- alternative treatment can be administered to the subject with marker-negative tumor.

8. The method of claim 6, wherein the specific treatment regimen is as follows:
- additional adjuvant therapy targeting specifically oncogenic/protective components of the IFN/STAT signaling pathway can be administered to the subject with marker-positive tumor.

9. The method of claim 8, wherein the additional adjuvant therapy is a PARP inhibitor.

10. The method of any of claims 6 to 9, wherein the biomarker is evaluated in tumor tissue obtained from a patient treated with chemotherapy between 24h and 21 days following the start of the treatment.

11. The method of claims 6, 8-9, wherein the biomarker is evaluated in a sample of cancer cells obtained from residual tumor tissue present in the surgical specimen obtained from a patient that received neoadjuvant chemotherapy.

12. A process for detection *in vitro* of at least one of the differential expressed biomarkers selected among the group consisting of biomarkers which are regulated by the IFN/STAT signaling pathway, expressed by human tumor cells after treatment by at least one chemotherapeutic drug comprising contacting said human tumor cell with a reagent capable of detecting said biomarker, wherein the differential expression is evaluated on the same tumor, between untreated tumor and post-treated tumor.

13. The process of claim 12, wherein the biomarker is evaluated in sample of cancer cells obtained from a patient treated with chemotherapy between 24h and 21 days following the start of the chemotherapy

14. The process of claim 13, wherein increased expression being predictive of sensitivity and response to chemotherapy, whereas lack of increase in expression being predictive of resistance or lack of response to chemotherapy.

15. The process of claim 12, wherein the biomarker is evaluated in sample of cancer cells obtained from residual tumor tissue present in the surgical specimen obtained from a patient that received neoadjuvant chemotherapy.

16. The process of claim 15, wherein differential expression of the biomarker is associated with cancer relapse following chemotherapy.

17. The process of any of claims 12 to 16, wherein the reagent is a nucleic acid probe that selectively binds to a nucleic acid encoding said biomarker.

18. The process of any of claims 12 to 16, wherein the reagent is an antibody molecule that binds selectively to the biomarker.

19. A method for analyzing differential expression of biomarker comprising the steps of: (i) measuring the amount of biomarker expression present in a tumor sample derived from a subject, and determining a sample value corresponding to said amount wherein said biomarker is selected among the group consisting of biomarkers which are regulated by the IFN/STAT signaling pathway, (ii) comparing the sample value obtained in step (i) with a reference value, said reference value being the one of the same biomarker in the same tumor prior to chemotherapy.

20. The method of claim 19, wherein the biomarker is evaluated in sample of cancer cells obtained from a patient treated with chemotherapy between 24h and 21 days following the start of the chemotherapy.

21. The method of claim 20, wherein increased expression being predictive of sensitivity and response to chemotherapy, whereas lack of increase in expression

22. The method of claim 19, wherein the biomarker is evaluated in sample of cancer cells obtained from residual tumor tissue present in the surgical specimen obtained from a patient that received neoadjuvant chemotherapy.

23. The method of claim 22, wherein differential expression is associated with cancer relapse following chemotherapy.

24. The method of any of claims 1 to 10, 19 to 23 or the process of any of claims 11 to 18, wherein the biomarker is one or more of the following biomarkers the expression of which is predictive of tumor sensitivity to a drug used during chemotherapy: PARP12, IFI44L, IFIT2, LAMP3, OAS2, PARP9, CCL5, IFIT1, OASL, STAT2, OAS1, IRF9, HERC6, UBE2L6, BST2, MX1, IFIT3, IFI44, DDX60, SP100, IFI6, SOD2, STAT1, RSAD2, IFIT5, C1R, OAS3, PARP14, PLSCR1, TLR3, DDX58, SAMHD1, MUC1, TRIM21, DTX3L, SAMD9, ZNFX1, IFI35, IFI27, IFITM1, MUC15, USP18, SAMD9L, TDRD7, C3, SP110, TLR2, DDIT3, HERC5, C1S, TRIM22, IFIH1, B2M, APOL6 and HLA-E.

25. The method of any of claims 1 to 10, 19 to 23 or the process of any of claims 11 to 18, wherein the biomarker is one or more of the following biomarkers the expression of which is predictive of tumor sensitivity to a drug used during chemotherapy: PARP12, DTX3L, CCL5, IFIT1, IFITM1, IRF9, IFI6, IFI44, IFI44L, OAS1, OAS2, LAMP3, MX1, PARP9, PARP14, SAMD9, SAMD9L, BST2, DDX60, CLDN1, STAT1, STAT2, UBE2L6, ZNFX1.
